(19) Europäisches Patentamt / European Patent Office / Office européen des brevets

(11) **EP 3 084 429 B1**

(12) **EUROPEAN PATENT SPECIFICATION**

(45) Date of publication and mention
of the grant of the patent:
**10.07.2019 Bulletin 2019/28**

(21) Application number: **14809403.0**

(22) Date of filing: **10.12.2014**

(51) Int Cl.:
*G01N 33/86* (2006.01)

(86) International application number:
**PCT/EP2014/077096**

(87) International publication number:
**WO 2015/091115 (25.06.2015 Gazette 2015/25)**

(54) **METHOD FOR DETERMINING THE HEMOSTATIC RISK OF A SUBJECT**

VERFAHREN ZUR BESTIMMUNG DES HÄMOSTATISCHEN RISIKOS EINER PERSON

PROCÉDÉ DE DÉTERMINATION DU RISQUE D'UN SUJET HÉMOSTATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2013 EP 13198633**

(43) Date of publication of application:
**26.10.2016 Bulletin 2016/43**

(73) Proprietor: **Koninklijke Philips N.V.**
**5656 AE Eindhoven (NL)**

(72) Inventors:
• **BAKKER, Bart Jacob**
**NL-5656 AE Eindhoven (NL)**
• **VAN OOIJEN, Hendrik Jan**
**NL-5656 AE Eindhoven (NL)**
• **VAN DEN HAM, Rene**
**NL-5656 AE Eindhoven (NL)**

(74) Representative: **Philips Intellectual Property & Standards**
**High Tech Campus 5**
**5656 AE Eindhoven (NL)**

(56) References cited:
**WO-A1-2012/172481     WO-A1-2012/172497**
**WO-A2-2009/142744**

- **HEMKER H C ET AL: "THROMBIN GENERATION IN PLASMA: ITS ASSESSMENT VIA THE ENDOGENOUS THROMBIN POTENTIAL", THROMBOSIS AND HAEMOSTASIS, SCHATTAUER GMBH, DE; US, vol. 74, no. 1, 1 July 1995 (1995-07-01), pages 134-138, XP000195941, ISSN: 0340-6245 cited in the application**
- **N. MACKMAN: "The many faces of tissue factor", JOURNAL OF THROMBOSIS AND HAEMOSTASIS, vol. 7, no. s1, 1 July 2009 (2009-07-01), pages 136-139, XP055169028, ISSN: 1538-7933, DOI: 10.1111/j.1538-7836.2009.03368.x**
- **N. MACKMAN: "Role of Tissue Factor in Hemostasis, Thrombosis, and Vascular Development", ARTERIOSCLEROSIS, THROMBOSIS, AND VASCULAR BIOLOGY, vol. 24, no. 6, 1 June 2004 (2004-06-01), pages 1015-1022, XP055169029, ISSN: 1079-5642, DOI: 10.1161/01.ATV.0000130465.23430.74**
- **KYRLE P A ET AL: "Deep vein thrombosis", THE LANCET, LANCET LIMITED. LONDON, GB, vol. 365, no. 9465, 26 March 2005 (2005-03-26), pages 1163-1174, XP027764608, ISSN: 0140-6736 [retrieved on 2005-03-26] cited in the application**

**Description**

FIELD OF THE INVENTION

The present invention relates to clinical decision support systems. In detail, the present invention relates to a computer-implemented method for determining the hemostatic risk of a subject, to a device for determining the hemostatic risk of a subject, to a computer program comprising a program code for carrying out the method for determining the hemostatic risk of a subject, and to a computer-readable non-transitory storage medium containing instructions for carrying out the method for determining the hemostatic risk of a subject.

BACKGROUND OF THE INVENTION

Deep vein thrombosis is a wide spread problem in the western world (Kyrle PA, Eichinger S. Deep vein thrombosis. Lancet. 2005;365(9465):1163-74). Large portions of the population run an increased risk of thrombosis, e.g. the elderly (Engbers MJ, van Hylckama Vlieg A, Rosendaal FR. Venous thrombosis in the elderly: incidence, risk factors and risk groups. J Thromb Haemost. 2010;8(10):2105-12), people who travel (Cannegieter SC, Doggen CJ, van Houwelingen HC, Rosendaal FR. Travel-related venous thrombosis: results from a large population-based case control study (MEGA study). PLoS Med. 2006;3(8):e307), and patients that undergo orthopaedic surgery (Kearon C, Kahn SR, Agnelli G, Goldhaber S, Raskob GE, Comerota AJ; American College of Chest Physicians. Antithrombotic therapy for venous thromboembolic disease: American College of Chest Physicians Evidence-Based Clinical Practice Guidelines (8th Edition). Chest. 2008 Jun; 133(6 Suppl):454S-545S). People at risk can be put on preventive anticoagulant treatment, but the risk of bleeding (1-3% per year) (Veeger NJ, Piersma-Wichers M, Tijssen JG, Hillege HL, van der Meer J. Individual time within target range in patients treated with vitamin K antagonists: main determinant of quality of anticoagulation and predictor of clinical outcome. A retrospective study of 2300 consecutive patients with venous thromboembolism. Br J Haematol. 2005;128(4):513-9), and issues of cost and inconvenience (Cohen AT, Tapson VF, Bergmann JF, Goldhaber SZ, Kakkar AK, Deslandes B, Huang W, Zayaruzny M, Emery L, Anderson FA Jr; ENDORSE Investigators. Venous thromboembolism risk and prophylaxis in the acute hospital care setting (ENDORSE study): a multinational cross-sectional study. Lancet. 2008;371(9610):387-94), speak against this. It would therefore be desirable to have a patient specific measure to estimate the personal thrombosis risk and facilitate an informed choice on whether or not to treat.

Unfortunately, with current clinical screening techniques and available methodologies, high risk individuals are not easily recognized and events are not accurately predicted (White RH. The epidemiology of venous thromboembolism. Circulation. 2003;107(23 Suppl 1):I4-8). One of the main reasons that this continues to be the case is that the vast majority of patients who suffer from thrombosis, those without obvious genetic defects, have blood coagulation systems that are not clinically identified as abnormal by routine screening tools and factor assays. Identification of individuals who are at risk for venous thrombosis is an area of research that could benefit from innovative technical methods (Brummel-Ziedins KE, Orfeo T, Rosendaal FR, Undas A, Rivard GE, Butenas S, Mann KG. Empirical and theoretical phenotypic discrimination. J Thromb Haemost. 2009;7 (Suppl 1):181-6).

The coagulation system has been the topic of extensive research in the past century. The sub-system that has been studied in the most detail is the coagulation cascade. This cascade describes the coagulation process from exposure of the blood to tissue factor (a protein that is normally shielded beneath the vessel wall, but triggers coagulation when exposed) to the production of thrombin, a key protein in the clotting process. The production of thrombin is well captured in the Thrombin Generation Assay (TGA) (Hemker, HC and Béguin, S. Thrombin generation in plasma: Its assessment via the endogenous thrombin potential, Thrombosis and haemostasis, vol. 74, no. 1, pp. 134-138, 1995), which measures thrombin concentration over time, after addition of a known concentration of tissue factor to a blood sample. Several features of the TGA, like lag time (time between the tissue factor trigger and occurrence of the first non-zero concentrations of thrombin), maximum thrombin concentration, time to maximum, maximum generation rate and endogenous thrombin potential (ETP, area under the plotted thrombin curve over time) have been tentatively linked to thrombosis risk.

Research (Jordan, SW and Chaikov, EL. Simulated Surface-Induced Thrombin Generation in a Flow Field. Biophysical Journal, Volume 101, July 2011 276-286) has shown that thrombin generation grows stronger (higher TGA maximum, shorter lag times) with increasing concentrations of tissue factor. For low (~1 fM) concentrations of tissue factor, no significant thrombin generation occurs, whereas for concentrations in the pM range it does. Based on this, the concept of a tissue factor threshold, i.e. a minimum concentration of tissue factor required for coagulation to start, has been hypothesized and observed (Mann, KG, Butenas, S. and Brummel, K. The Dynamics of Thrombin Formation. Arterioscler. Thromb. Vasc. Biol. 2003;23:17-25, Jordan and Chaikov, l.c.).

Uncertainty about the patient specific risk of thrombosis causes unnecessary cases of thrombosis in patients at high risk (of thrombosis) who do not receive anticoagulant treatment. On the other hand, this uncertainty can result in bleeding in patients at relatively low risk who do receive unnecessary anticoagulant treatment. The current state of the art (Hippisley-Cox, J and Coupland, C. Development and validation of risk prediction algorithm (QThrombosis) to estimate future risk of venous thromboembolism: prospective cohort study, BMJ 2011; 343) estimates thrombosis risk based on a number of clinical risk factors. This is however not sufficiently specific.

Documents US 2009/0298103 and WO 2009/142744 disclose a method for determining a hemostatic risk in a patient by subjecting the concentrations of various blood factors to a computer model. With this model the thrombin concentrations are simulated and the simulated concentrations are compared to a reference. According to the authors, the comparison allows a decision of a clinician whether the patient is predisposed to a hemostatic risk. However, such method has not proven its value in the clinical practice.

SUMMARY OF THE INVENTION

It is an object of the invention to provide a method for determining the hemostatic risk of a subject by means of which the disadvantages of the prior art methods can be avoided. In particular, such a method for determining the hemostatic risk of a subject should be provided which allows a reliable diagnosis on whether a subject has a high risk of thrombosis and might require anti-coagulation medication or whether it has a high risk of bleeding contra-indicating or even requiring a stop of anti-coagulation medication.

It is another object of the invention to provide a device for determining the hemostatic risk of a subject, a computer program comprising a program code for carrying out a method for determining the hemostatic risk of a subject, and a computer-readable non-transitory storage medium containing instructions for carrying out a method for determining the hemostatic risk of a subject.

In a first aspect of the present invention a computer-implemented method for determining a hemostatic risk of a subject is presented, the method comprising a comparison of a concentration value of a clotting trigger sufficient to start the clotting process in a subject with at least one reference concentration value of said clotting trigger indicative for a hemostatic risk and/or a hemostatic non-risk (hemostatic stability), wherein the method comprises the steps of:

a) providing a first information on the hemostatic condition of said subject, wherein said first information is the concentration values of a plurality of coagulation proteins in a blood sample from said subject,

b) determining on the basis of said first information of step (a) a concentration value of a clotting trigger sufficient to start the clotting process in said subject, wherein said clotting trigger is tissue factor (TF), wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,

c) comparing said concentration value determined in step (b) with a reference concentration value of said TF representing in healthy reference subjects the minimum concentration for a stable hemostatic condition, and

d) determining a high risk of thrombosis for said subject if said concentration value determined in step (b) is lower than said reference concentration value of said TF representing in healthy reference subjects the minimum concentration for a stable hemostatic condition.

In another aspect the present disclosure is directed to the use of a concentration value of a clotting trigger sufficient to start the clotting process in a subject for determining the hemostatic risk of said subject.

The method according to the invention is realized *in vitro,* i.e. the physical presence of the subject, such as a living or human being, is not necessary. For doing so, the concentration value of a clotting trigger sufficient to start the clotting process can be measured or generated in a step prior to the method or use according to the invention.

In still another aspect of the present invention a device is provided for determining the hemostatic risk of a subject, said device comprising:

a receiving unit configured for receiving a first information on the hemostatic condition of said subject wherein said first information is the concentration values of a plurality of coagulation proteins in a blood sample from said subject, a first determining unit configured for determining on the basis of said first information received by the receiving unit a concentration value of a clotting trigger sufficient to start the clotting process in said subject, wherein said clotting trigger is tissue factor (TF), wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,
a comparing unit configured for comparing said concentration value determined by said first determining unit with at least one reference concentration value of said TF indicative in healthy reference subjects for a hemostatic risk and/or a hemostatic non-risk (hemostatic stability), wherein said comparing unit is configured for comparing said concentration value determined by said first determining unit with a reference concentration value of said TF representing in healthy reference subjects the minimum concentration for a stable hemostatic condition, and said device is further comprising:
a second determining unit configured for determining a high risk of thrombosis for said subject if said concentration value determined by said first determining unit is lower than said reference concentration value of TF representing in healthy reference subjects the minimum concentration for a stable hemostatic condition.

In still another aspect of the present invention a clinical decision support system is presented, said system comprising a processor and a computer-readable storage medium, wherein said computer-readable storage medium contains instructions for execution by the processor, wherein said instructions cause said processor to perform the steps of:

a) receiving a first information on the hemostatic condition of a subject wherein said first information is the concentration values of a plurality of coagulation proteins in a blood sample from said subject,
b) determining on the basis of said information of step (a) a concentration value of a clotting trigger sufficient to start the clotting process in said subject, wherein said clotting trigger is tissue factor (TF), wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,
c) comparing said concentration value determined in step (b) with at least one reference concentration value of said TF indicative in healthy reference subjects for a hemostatic risk and/or a hemostatic non-risk (hemostatic stability).

In still another aspect of the present invention a clinical decision support system is presented, said system comprising a processor and a computer-readable storage medium, wherein said computer-readable storage medium contains instructions for execution by the processor, wherein said instructions cause said processor to perform the steps of:

a) providing a first information on the hemostatic condition of said subject wherein said first information is the concentration values of a plurality of coagulation proteins in a biological sample from said subject,
b) determining on the basis of said first information of step (a) a concentration value of a clotting trigger sufficient to start the clotting process in said subject wherein said clotting trigger is tissue factor (TF), wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,
c) comparing said concentration value determined in step (b) with a reference concentration value of said TF representing in healthy reference subjects the minimum concentration for a stable hemostatic condition, and representing in healthy reference subjects the minimum concentration for a stable hemostatic condition, and
d) determining a high risk of thrombosis for said subject if said concentration value determined in step (b) is lower than said reference concentration value of said TF representing in healthy reference subjects the minimum concentration for a stable hemostatic condition.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed system, device, computer program and medium have similar and/or identical preferred embodiments as the claimed method and as defined in the dependent claims. Therefore, all of the dependent claims referring to the method according to the invention can also be combined with the system, device, computer program and medium according to the invention and with each other.

The proposed invention significantly increases the accuracy of a person's specific thrombosis and bleeding risk estimation, especially within the increased risk subgroup of patients with at least one known clinical risk factor present. This subgroup involves - among others - patients that are hospitalized, are pregnant or are (start) using oral contraceptives and thus receive attention of a physician. In this context, the proposed invention helps the physician to stratify the patients that are treated or examined for conditions that are known to increase thrombosis risk, into high and low risk categories. Specifically, the improved method may be used to decide, per patient, whether or not to administer anticoagulant treatment based on estimated thrombosis or bleeding risk.

In contrast to the method known from prior art documents US 2009/0298103 and WO 2009/142744 where thrombin as the key factor of the coagulation process is simulated *in silico* the invention does use a different approach. In the invention a clotting trigger is determined, i.e. a factor that initiates the coagulation process, rather than the coagulation process itself. This approach allows a more precise and reliable prediction of the hemostatic risk and does not necessitate a complex and error-prone simulation *in silico* of the thrombin formation like with the prior art method.

According to the invention "the hemostatic risk" refers to the risk of a subject, such as a human or animal being, of having a dysfunction of the haemostasis which might result in the development of thrombosis or bleeding.

According to a further development of the method according to the invention steps c) and d), additionally, comprise the following steps:

c') comparing said concentration value determined in step (b) with a reference concentration value of said TF representing in healthy reference subjects the maximum concentration for a stable hemostatic condition, and

d') determining a high risk of bleeding for said subject if said concentration value determined in step (b) is higher than said reference concentration value of said TF representing in healthy reference subjects the maximum concentration for a stable hemostatic condition.

In a further development of the device according to the invention, additionally,

said comparing unit is configured for comparing said concentration value determined by said first determining unit with a reference concentration value of said TF representing in healthy reference subjects the maximum concentration for a stable hemostatic condition, and

said second determining unit is configured for determining a high risk of bleeding for said subject if said concentration value determined by said first determining unit is higher than said reference concentration value of said TF representing in healthy reference subjects the maximum concentration for a stable hemostatic condition.

In still another aspect of the present invention a clinical decision support system is presented, said system comprising a processor and a computer-readable storage medium, wherein said computer-readable storage medium contains instructions for execution by the processor, wherein said instructions cause said processor to perform the steps of:

a) receiving a first information on the hemostatic condition of a subject, wherein said first information is the concentration values of a plurality of coagulation proteins in a blood sample from said subject,

b) determining on the basis of said information of step (a) a concentration value of a clotting trigger sufficient to start the clotting process in said subject, wherein said clotting trigger is tissue factor (TF), wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,

c) comparing said concentration value determined in step (b) with a reference concentration value of said TF representing in healthy reference subjects the minimum concentration for a stable hemostatic condition, and

d) determining a high risk of thrombosis for said subject if said concentration value determined in step (b) is lower than said reference concentration value of said TF representing in healthy reference subjects the minimum concentration for a stable hemostatic condition,

wherein preferably, steps c) and d) additionally comprise the following steps:

c') comparing said concentration value determined in step (b) with a reference concentration value of said TF representing in healthy reference subjects the maximum concentration for a stable hemostatic condition, and
d') determining a high risk of bleeding for said subject if said concentration value determined in step (b) is higher than said reference concentration value of said TF representing in healthy reference subjects the maximum concentration for a stable hemostatic condition.

As described herein, a "clotting trigger" refers to a factor being present in the subject's body liquid, such as blood, being capable of initiating the coagulation process in the subject. The coagulation process is initiated when the clotting trigger reaches a critical concentration in the subject's body liquid. According to the invention said critical concentration is referred to the "concentration value sufficient to start the clotting process in said subject" or "clotting trigger threshold". The clotting trigger used in the present invention is tissue factor (TF) (extrinsic pathway).

According to the invention in step (b) such critical concentration of the clotting trigger is determined by employing a computer simulation which takes the information of the hemostatic condition of the subject, namely concentration values of coagulation proteins from the subject's blood sample (without the clotting trigger) as inputs, and calculating the clotting response depending on the clotting trigger concentrations.

A "reference concentration value of said clotting trigger" refers to a value of the clotting trigger which has been obtained from one or a plurality of reference subjects or reference *in silico* calculations. The reference concentration can be the result of an individual measurement or calculation or an average value of a plurality of measurements or calculations, respectively.

According to the invention the "minimum/maximum concentration for a stable hemostatic condition" refers to concentration values of the clotting trigger limiting a concentration range of said clotting trigger which can be found in healthy reference subjects characterized by a functional, stable hemostasis ('healthy range'). The minimum concentration of the clotting trigger limits the healthy range at its lower boundary; the maximum concentration of the clotting trigger limits the healthy range at its upper boundary. Alternatively, said minimum and maximum concentrations might refer to concentration values of the clotting trigger limiting a concentration range of said clotting trigger which can be found in patient subjects on anticoagulants ('therapeutic range'). Both ranges can be inferred from literature or determined in a targeted patient study.

Said "concentration value of a clotting trigger sufficient to start the clotting process" is also referred to as a "clotting trigger threshold". For concentrations of the clotting trigger below that threshold the clotting response remains at a minimum, for concentrations of the clotting trigger above the threshold the clotting response starts to rise. In steps (c) and (c') the clotting trigger threshold is compared with the boundary values of the 'healthy range'.

According to the invention, if the clotting trigger threshold as determined in step (b) falls below the 'healthy range' the subject is deemed to be at high risk of thrombosis as diagnosed in step (d) and anti-coagulant treatment might be indicated. If the threshold falls above the range, the patient is deemed to be at high risk of serious bleeding as diagnosed in step (d') and anti-coagulant use might be counter-indicated or may even be stopped if the patient was using anticoagulants at the time of testing.

The object underlying the invention is herewith fully achieved. The inventors have surprisingly realized that a concentration value of the tissue factor (TF) sufficient to start the clotting process in a subject can be used for determining the hemostatic risk of said subject. Even though the notion of a 'tissue factor threshold' exists and has been mentioned in literature the use of this threshold as a way to indicate thrombosis or bleeding risk is neither disclosed nor rendered obvious by the prior art. Furthermore, in the art the concept of a 'tissue factor threshold' has always been linked to visual signs of clotting, e.g. solidifying of the blood sample or thrombin generation. The use of other coagulation features, which often manifest themselves before thrombin generation or formation of the clot, is made possible by the method according to the invention and may provide an improvement over thrombin based methods in the art.

According to an alternative embodiment of the invention the method comprises the following steps:

a) providing a first information on the hemostatic condition of said subject (S1), wherein said first information is the concentration values of a plurality of coagulation proteins in a blood sample from said subject,
b) determining on the basis of said first information of step (a) a concentration value of a clotting trigger sufficient to start the clotting process in said subject (S2), wherein said clotting trigger is tissue factor (TF), wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,
c) comparing said concentration value determined in step (b) with at least two or more reference concentration values of said TF comprising at least one reference concentration value indicative in healthy reference subjects for a hemostatic risk, preferably a high risk of thrombosis and/or a high risk of bleeding, and at least one reference concentration value indicative in healthy reference subjects for a hemostatic non-risk (hemostatic stability) (S3"), and
d) determining

- a hemostatic risk for said subject if said concentration value determined in step (b) is numerically closer to said at least one reference concentration value indicative in healthy reference subjects for a hemostatic risk (S4"), or
- a hemostatic non-risk (hemostatic stability) for said subject if said concentration value determined in step (b) is numerically closer to said at least one reference concentration value indicative in healthy reference subjects for a hemostatic stability (S4'").

In this alternative approach results the clotting trigger threshold is compared with (different) reference values in view of being numerically closest to one of the latter. This results in a gliding scale rather than a binary output. It is preferred that steps (c) and (d) are realized by means of a nearest neighbor approach or a nearest-neighbor interpolation, respectively.

According to that alternative embodiment of the invention a device for determining the hemostatic risk of a subject is provided, said device comprises:

a receiving unit configured for receiving a first information on the hemostatic condition of said subject, wherein said first information is the concentration values of a plurality of coagulation proteins in a blood sample from said subject, a first determining unit configured for determining on the basis of said first information received by the receiving unit a concentration value of a clotting trigger sufficient to start the clotting process in said subject, wherein said clotting trigger is tissue factor (TF), wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF, and a comparing unit configured for comparing said concentration value determined by said first determining unit with at least two or more reference concentration values of said TF comprising at least one reference concentration value indicative in healthy reference subjects for a hemostatic risk, preferably a high risk of thrombosis and/or a high risk of bleeding, and at least one reference concentration value indicative in healthy reference subjects for a hemostatic non-risk (hemostatic stability), and a second determining unit configured for determining

- a hemostatic risk for said subject if said concentration value determined by said first determining unit is numerically closer to said at least one reference concentration value indicative in healthy reference subjects for a hemostatic risk, or
- a hemostatic non-risk (hemostatic stability) for said subject if said concentration value determined by said first determining unit is numerically closer to said at least one reference concentration value indicative in healthy reference subjects for a hemostatic stability.

Consequently, in another aspect of the invention a clinical decision support system is provided comprising a processor and a computer-readable storage medium, wherein said computer-readable storage medium contains instructions for execution by the processor, wherein said instructions cause said processor to perform the steps of:

a) providing a first information on the hemostatic condition of said subject, wherein said first information is the concentration values of a plurality of coagulation proteins in a blood sample from said subject,
b) determining on the basis of said first information of step (a) a concentration value of a clotting trigger sufficient to start the clotting process in said subject, wherein said clotting trigger is tissue factor (TF), wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,

c) comparing said concentration value determined in step (b) with at least two or more reference concentration values of said TF comprising at least one reference concentration value indicative in healthy reference subjects for a hemostatic risk, preferably a high risk of thrombosis and/or a high risk of bleeding, and at least one reference concentration value indicative in healthy reference subjects for a hemostatic non-risk (hemostatic stability), and
d) determining

- a hemostatic risk for said subject if said concentration value determined in step (b) is numerically closer to said at least one reference concentration value indicative in healthy reference subjects for a hemostatic risk, or
- a hemostatic non-risk (hemostatic stability) for said subject if said concentration value determined in step (b) is numerically closer to said at least one reference concentration value indicative in healthy reference subjects for a hemostatic stability.

In step (a) said first information on the hemostatic condition of said subject is the concentration values of a plurality of coagulation proteins in a biological sample from said subject, preferably in step (a) said first information on the hemostatic condition of said subject is the concentration values of at least three or more coagulation proteins in a biological sample from said subject.

These measures have the advantage that such information on the hemostatic condition of a subject is used for realizing the method according to the invention which has been proven as being appropriate for the determination of the clotting trigger threshold. Preferably, in this embodiment the coagulation proteins are present in their inactivated form. The concentration values of the coagulation proteins can be measured for said specific subject or average values taken from literature can be used. Alternatively or additionally, the concentration values measured for said specific subject and combined with the average values taken from literature for those coagulation proteins which are not measured in said specific subject. According to the invention "biological sample" refers to blood.

According to the invention said clotting trigger is the tissue factor (TF).

This measure has the advantage that such a clotting trigger is employed which is thought to be the main trigger of coagulation and has been proven as being particularly suited for the realization of the invention.

According to a further aspect said coagulation protein(s) is/are selected from the group consisting of: coagulation factor 2 (FII), FV, FVII, FVIII, FIX, FX, FXI, FXII, antithrombin (AT), TFPI, $\alpha$2M, C4BP, protein C, protein S, protein Z, TAFI, ZPI, AAT, PCI, C1 inhibitor and fibrinogen.

This further development of the invention has the advantage that such (inactivated) coagulation proteins are used to calculate the clotting trigger threshold which, according to the findings of the inventors, produce notably good and reliable results.

Pursuant to the invention in step (b) said concentration value of a clotting trigger sufficient to start the clotting process in the subject is determined by an *in silico* simulation of the clotting process.

The clotting trigger could be determined *in situ* or *vitro,* e.g. by visually examining signs of clotting or solidifying of the blood sample, respectively, or thrombin generation. The use of other coagulation features, which often manifest themselves before thrombin generation or formation of the clot, is made possible by the use of a computer model of the coagulation process. Such *in silico* simulation may provide an improvement over thrombin based methods.

According to the invention in the *in silico* simulation said first information on the hemostatic condition of said subject, namely concentration values of (inactivated) coagulation proteins, is used as input feature, and a second information on the hemostatic condition of said subject in the simulated clotting process is used as output feature.

According to the invention said second information on the hemostatic condition of said subject is the concentration values of an activated coagulation protein at a series of time points of the simulated clotting process which are used as a set of output features, preferably said second information on the hemostatic condition of said subject is the concentration values of a plurality of activated coagulation proteins at a series of time points of the simulated clotting process which are used as a set of output features. However, it is to be understood that, even the activated coagulation proteins are preferred, in principle the concentration values of non-activated coagulation proteins can be used as well. If, e.g., the concentration values of non-activated coagulation proteins are known over time, such as FX, also the concentration values of the produced activated counterpart are known, i.e. FXa, since this is the initial concentration of FX minus the concentrations of FX at later stages.

In comparison with a determination of the clotting trigger threshold *in vitro* or *in situ* a much wider range of features can be simulated in a computer simulation which takes measured protein concentrations from a patient's sample, such as a blood sample (without tissue factor trigger) and/or average values from literature as inputs and calculates the clotting response and the clotting trigger threshold, respectively. Therefore, such measure significantly increases the accuracy of the diagnosis of the subject's specific risk of thrombosis or bleeding.

According to a further development of the invention said activated coagulation protein(s) is/are selected from the group consisting of: thrombin (FIIa), FVa, FVIIa, FVIIIa, FIXa, FXa, FXIIa, FVa-FXa, FVIIIa-FIXa, fibrin, prothrombin (FII).

This measure has the advantage that any of the afore-mentioned activated coagulation proteins has been proven as being suitable to determine the clotting trigger threshold.

Said second information on the hemostatic condition of said subject can also be embodied by the endogenous thrombin potential (ETP). The ETP is defined as the time integral or the area under the curve of the thrombin concentration over the course of the simulation and is often related to the amount of fibrin which can be generated after the in vitro activation of coagulation with tissue factor as trigger and phospholipids as platelet substitute.

According to another aspect of the invention out of the set of output features one feature is created representing the strength of the clotting response, preferably said one feature representing the strength of the clotting response is the maximum concentration of at least one of said activated coagulation proteins over all time points of the simulated clotting process or the ETP (time integral over an activated protein (e.g. FIIa) or total production of thrombin (i.e. $FII(t=0)-FII(t=t\_end)$, where $t=0$ denotes the time of first exposure to a clotting trigger and $t\_end$ denotes that time after first exposure (e.g. one hour) that is deemed to be such that the clotting process takes place completely within the interval $t=0$ to $t=t\_end$).

This measure has the advantage that a feature is used which allows the *in silico* determination of the clotting trigger threshold. In the simulation the clotting response e.g. the maximum concentration of a specific activated coagulation protein, such as F10a, is used, or a variety of activated coagulation proteins, to calculate the concentration value of the clotting trigger, e.g. of TF, sufficient to start the clotting process.

It shall be understood that a preferred embodiment of the invention can also be any combination of the dependent claims with the respective independent claim.

These and other aspects of the invention will be apparent from and elucidated with reference to the embodiments described hereinafter.

BRIEF DESCRIPTION OF THE DRAWINGS

In the drawings:

Figure 1 shows a graph demonstrating the phenomenon of a clotting trigger threshold exemplified for the tissue factor;

Figure 2 shows a model output of concentrations of an activated coagulation protein, exemplified for F10a, at 100 points in time during the simulated coagulation or clotting process;

Figure 3 schematically shows a respective flow diagram of a method according to exemplary embodiments of the invention;

Figure 4 schematically shows a respective flow diagram of a method according to another exemplary embodiment of the invention

Figure 5 shows a bar diagram of the risk score evaluated on the basis of clinical risk factors (CRF), single nucleotide polymorphisms (SNPs), protein levels and model based thresholds in a cross-validation study on the MEGA database.

DETAILED DESCRIPTION OF EMBODIMENTS

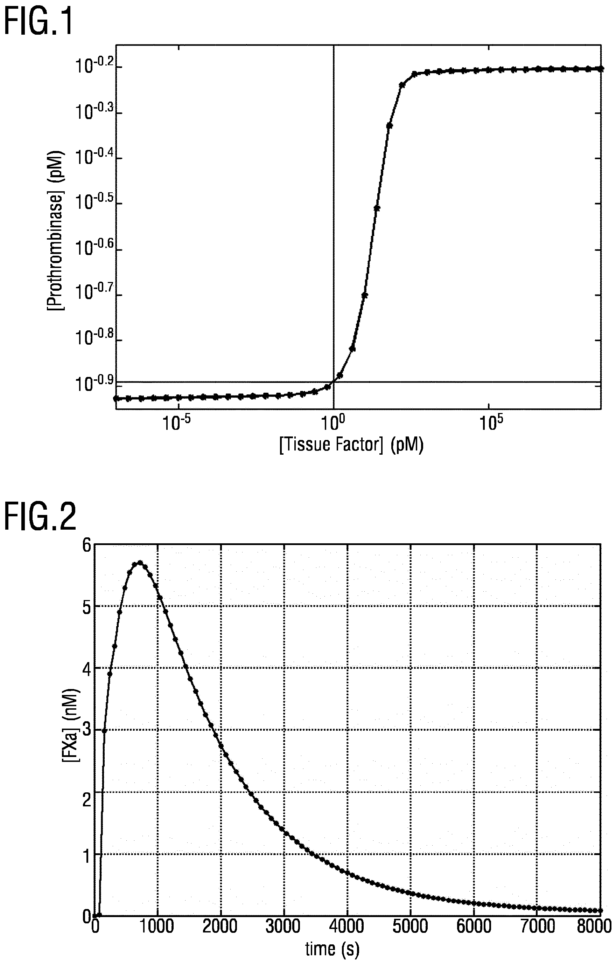

The invention identifies a person's expected clotting response at a series of clotting triggers, e.g. in the form of increasing concentrations of tissue factor, e.g. the main protein that initiates the clotting process. Reference is made to Fig. 1 giving an example for the phenomenon of a clotting trigger threshold exemplified for the tissue factor. Each star in the curve represents a computer simulation. The input of each simulation consists of the measured coagulation protein concentrations in a patient's blood sample, along with literature values for the unmeasured concentrations. The x-axis represents the concentrations of tissue factor that are used as the final model input. The y-axis represents one chosen feature from the simulations' output, in this case the maximum concentration of prothrombinase (FVa-FXa). It can be seen that for low concentrations of tissue factor the clotting response remains at a minimum. At the tissue factor threshold, indicated by the vertical line, the clotting response starts to rise visibly and the chosen model output feature exceeds the chosen start-off level, indicated by the horizontal line. For sufficiently large tissue factor concentrations the response levels off again.

The clotting response (the y-axis of Figure 1) can be taken as any representative feature in the clotting process. Commonly studied features (in a research setting) are thrombin generation features like maximum thrombin concentration or lag time. Similar features can be used on the generation of other enzymes in the coagulation cascade like FXa or enzyme complexes like FVIIIa-FIXa. In a broader sense features like clot size can be used as well. Some such features can be measured *in vitro,* allowing an implementation in a laboratory environment. A much wider range of features can be simulated in a computer model which takes measured protein concentrations from a patient's blood sample (without tissue factor trigger) as inputs and calculated the clotting response for the aforementioned series of increasing tissue factor concentrations.

The numerical value of the tissue factor threshold (a concentration value) is recorded for a patient and compared to a pre-defined 'healthy range' or 'therapeutic range' for patients on anticoagulants. If the threshold falls below this range, the patient is deemed to be at high risk of thrombosis, and anti-coagulant treatment is indicated. If the threshold falls above the range, the patient is deemed to be at high risk of serious bleeding and anti-coagulant use is counter-indicated or may even be stopped (if the patient was using anticoagulants at the time of testing).

One embodiment of the invention involves the use of a computer model that is able to simulate the dynamic change in coagulation protein concentrations after exposure to tissue factor. The patient specific input of the model consists of concentration values of the proteins that play a role in the coagulation cascade before clotting. The model simulates a blood sample with these concentrations and a non-zero concentration of clotting trigger, e.g. tissue factor, at time t=0. The output of the model consists of the concentrations of all proteins and protein complexes that are involved in the coagulation process, for a series of time points $t_i > 0$.

One simulation thus generates $P * T$ numerical outputs, with $P$ the number of proteins and protein c complexes that are described in the model and $T$ the number of time points for which a model output is generated. Out of this set of outputs one feature is created that represents the strength of the coagulation response. An example of such a feature could be the maximum concentration of one protein, e.g. FXa, over all time points in the model output.

Reference is made to Figure 2 where the model output FXa concentration at 100 points in time (0-8000 seconds in 80 second intervals) is shown. The maximum concentration (5.7 nM in this graph) can be used as coagulation response feature. Note that the concentration features of the proteins in the coagulation cascade are strongly correlated, so many different model output features, or combinations of model output features can be selected as the coagulation response feature to be used in the determination of the tissue factor threshold.

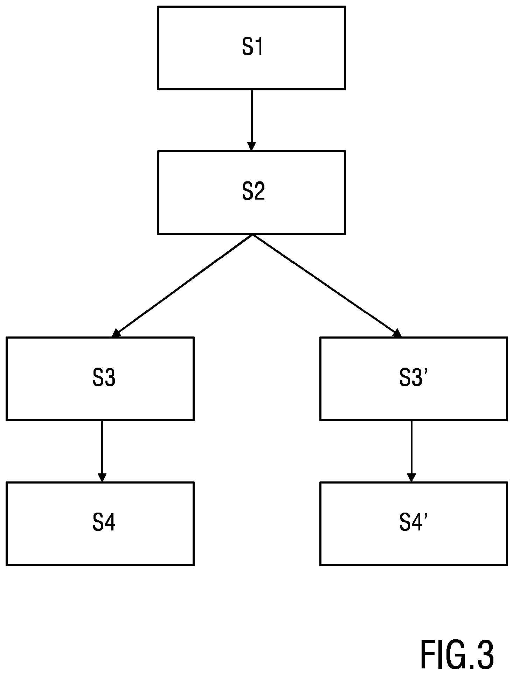

Figure 3 shows a flow diagram of an embodiment of the method for determining the hemostatic risk of a subject wherein the presented method comprises steps (S1) to (S4) and/or (S1) to (S4'), respectively.

In step (S1) a first information on the hemostatic condition of said subject is provided, such as concentration values of a plurality of coagulation proteins in a biological sample from said subject. Such information may be received by a receiving unit of a device for determining the hemostatic risk of a subject or a clinical decision support system. The concentration values may be measured values or average values taken from literature. In practice, a blood sample, e.g. finger prick sample, is taken from a person for whom the hemostatic risk, i.e. the risk of thrombosis or bleeding needs to be determined. The concentrations of a number of (inactivated) coagulation proteins are determined in this sample via standard methods like ELISA assays. The measured proteins are preferably one, more or all of the following: coagulation factor 2 (FII), FV, FVII, FVIII, FIX, FX, FXI, FXII, antithrombin (AT), TFPI, $\alpha$2M, C4BP, protein C, protein S, protein Z, TAFI, ZPI, AAT, PCI, C1 inhibitor and fibrinogen. In practice, the measurement of one or a subset (e.g. FII, FV, FVIII, FX, FXI, AT and fibrinogen) would be sufficient. Alternatively or in addition average values are taken from literature.

In step (2), on the basis of said first information of step (S1), a concentration value of a clotting trigger sufficient to start the clotting process in said subject is determined. Such determination might be realized by a determining unit of a device for determining the hemostatic risk of a subject or a clinical decision support system. In practice a computer model is used. With this model $N$ simulations are performed with the following inputs: For the proteins in the measured (sub) set, use the measured values. For the unobserved protein concentrations, use the literature or average values. These inputs, i.e. the concentrations of the P proteins at time zero (the time when the simulated blood is first exposed to a clotting trigger), are the same in each of the $N$ simulations. The concentration for the tissue factor varies between $TF_{min}$ for the first simulation and $TF_{max}$ for simulation $N$. For every different choice of tissue factor concentration, a response is calculated through the model. In other words, a model simulation is performed which describes the coagulation response that corresponds to the chosen tissue factor concentrations. A response consists of the development of the concentration of a certain protein over time, starting at the time of first exposure of the blood to the clotting factor, e.g. tissue factor (initiation of the wound), and ending at a preset length of time. The model calculates these dynamics for a number of different proteins; Figure 2 is an example for such protein where the response is limited to one characteristic quantity, e.g. the maximum concentration of FXa (the peak of the graph in Figure 2). The concentration for simulation $i$ may be chosen as

$$TF_i = TF_{min} + i * (TF_{max}\text{-}TF_{min}) \, / \, N, \qquad (1)$$

or

$$TF_i = exp( \, log(TF_{min)} + i * (log(TF_{max})\text{-}log(TF_{min})) \, / \, N \, ) \qquad (2)$$

for $i$ = 0..N, where formula (1) is most suitable when $TF_{min}$ and $TF_{max}$ are of the same order of magnitude, whereas formula (2) is more suitable for larger ranges of tissue factor concentrations. Example values are $TF_{min}$ = 1x10$^{-4}$ fM and $TF_{max}$ = 1 mM, with *N*=100. This large range (for which formula (2) is the most fitting) is certain to include the tissue factor threshold in all but the most extreme cases, and *N* is high enough to catch its value with high precision.

As such, every combination of protein concentrations and clotting trigger or tissue factor concentration (as in the first item), can be used to calculate one numerical feature (one number) through the model. Then these numerical features are calculated for N different tissue factor concentrations, whereas the proteins that were measured in the patient or taken from literature were kept constant (the same values in each of the N model simulations). This produces N numerical features; Figure 1 plots the value of one such feature on the Y-axis with the value for the corresponding tissue factor concentrations on the X-axis. The different values for the clotting trigger or tissue factor concentrations, respectively, start at a very low value, and increase with a certain step size up to a large value, such as to cover the range from the point where certainly no strong clotting response will take place, to the point where for sure a clotting response will take place. Formulae (1) and (2) describe these steps: Formula (1) describes a clotting trigger concentration exemplified for the minimum tissue factor concentration of $TF_{min}$ and the maximum tissue factor concentration of $TF_{max}$, with equal sized steps in between. The $TF_i$ are the tissue factor concentrations for which the model output is calculated. Formula (2) describes the same, only now the steps have equal size in the log domain (for example, if the regarded tissue factor concentrations would vary over multiple orders of magnitude, say between 1 and 1.000.000, it would be more reasonable to look at tissue factor concentrations of 1, 10, 100, ..., 1.000.000 than 1, 2, 3, ..., 1.000.000; the first type of steps is what one gets in the log10 domain).

Once the model output is calculated for all clotting trigger or tissue factor concentrations, respectively, one will have a graph like in Figure 1. By eye one can see where the response starts to rise. This point is referred to as the clotting trigger threshold or the tissue factor threshold, respectively. Such threshold can be calculated by the following algorithm:

Take the minimum Y-value in the graph of Figure 1 [min($Y_i$)] and the dynamic range, i.e. the difference between the highest and the lowest point in the graph [max($Y_i$) - min($Y_i$)]. It is hypnotized that the response starts to rise when it is more than 5% of the dynamic range higher than the minimum, leading to the formula:

$$\min(Y_i) + 0.05 * (\max(Y_i) - \min(Y_i)) \tag{3}$$

where $Y_i$ is the obtained value for the coagulation response feature in simulation *i*; where min($Y_i$) is the minimum concentration value of the activated coagulation protein over all simulations, and
where max($Y_i$) is the maximum concentration value of the activated coagulation protein over all simulations.

The horizontal line in Figure 1 is an example of such a start-off level. An interpolation technique (e.g. linear) is used to determine the tissue factor threshold, i.e. the concentration where the coagulation response curve exceeds the start-off level (see vertical line in Figure 1). The corresponding tissue factor threshold value for this patient is stored.

In step (S3) said concentration value or tissue factor threshold value, respectively, determined in step (S2) is compared with a reference concentration value of said clotting trigger representing the minimum concentration for a stable hemostatic condition. In addition or alternatively, in step (S3') said concentration value determined in step (S2) is compared with a reference concentration value of said clotting trigger representing the maximum concentration for a stable hemostatic condition. Such comparison might be realized by a comparing unit of a device for determining the hemostatic risk of a subject or a clinical decision support system. In practice, in (S3) the tissue factor threshold is compared to a 'minimum stable level' (to be determined in a targeted patient study). In addition or alternatively, in (S3') the tissue factor threshold can be compared to a 'maximum stable level'. The 'minimum stable level' and 'maximum stable level' can be taken from literature or determined in a targeted patients study.

In step (S4) a high risk of thrombosis for said subject is determined if said concentration value determined in step (S2) is lower than said reference concentration value of said clotting trigger representing the minimum concentration for a stable hemostatic condition. In step (S4') a high risk of bleeding for said subject is determined if said concentration value determined in step (S2) is higher than said reference concentration value of said clotting trigger representing the maximum concentration for a stable hemostatic condition. Such determining step might be realized by a determining unit of a device for determining the hemostatic risk of a subject or a clinical decision support system. In practice, if the patient's threshold is lower than the 'minimum stable level' level, in (S4) the patient is stratified as being 'at high risk of thrombosis'. If the patient's threshold exceeds this level, in (S4') the patient is stratified as being 'at high risk of bleeding'. The combination of the minimum and maximum level can indicate a stable region, which may have different boundaries for anti-coagulant users (therapeutic region) and non-coagulant users (healthy region). Based on the risk stratification in (S4) and/or (S4'), the clinician can decide whether or not to start anticoagulant treatment on a non-anticoagulated patient, or to stop anticoagulant treatment on a patient who does currently use anticoagulants.

Steps (S1) and (S2) can be replaced by *in vitro* measurements of a coagulation response for a series of increasing clotting trigger or tissue factor concentrations, respectively. This is however more expensive in terms of time (and in most cases money), limits the choice of coagulation response feature and such measurements are known to become highly unreliable for small clotting triggers, e.g. ~1 fM tissue factor. The advantage is that this embodiment does not rely on the quality of a computer model.

Furthermore, steps (S4) and/or (S4') may be replaced by a data-driven algorithm. Such an algorithm can be a neural network based algorithm, which combines known thrombosis risk factors such as recent surgery, immobilization or the FV Leiden genetic mutation with the value for the clotting trigger or tissue factor threshold, respectively, that was obtained in step (S2), and returns a thrombosis risk score between zero and one. A similar algorithm can be described for bleeding risk.

Steps (S1), (S2), (S3), and (S4) and (S1), (S2), (S3'), and (S4') can be seen as alternative routes. In case the risk of thrombosis is to be determined the first route ('thrombosis risk route'; left route in Figure 3) is used. In case the risk of bleeding is to be determined the second route ('bleeding risk route', right route in Figure 3) is used. However, steps (S3) and (S3') as well as steps (S4) and (S4') can be taken in parallel or additionally, i.e. in one method, allowing the subsequent or parallel determination of the subject's risk of thrombosis or bleeding, e.g. by one device. It is also possible to go through the 'thrombosis risk route' and in case of a negative outcome to successively go through the 'bleeding risk route', i.e. (S1), (S2), (S3), (S4), (S3'), (S4'), or vice versa, i.e. (S1), (S2), (S3'), (S4'), (S3), (S4).

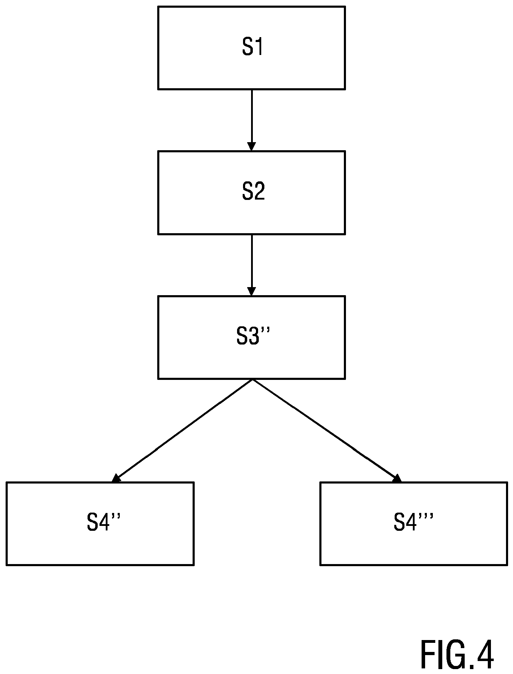

An alternative approach is depicted in Fig. 4. Steps (S1) and (S2) are identical with the approach shown in Fig. 3. However, in the next step (S3") said concentration value determined in step (S2) is compared with at least two or more reference concentration values of said clotting trigger comprising at least one reference concentration value indicative for a hemostatic risk, preferably a high risk of thrombosis and/or a high risk of bleeding, and at least one reference concentration value indicative for a hemostatic non-risk (hemostatic stability). In step (S4") a hemostatic risk for said subject is determined if said concentration value determined in step (S2) is numerically closer to said at least one reference concentration value indicative for a hemostatic risk. In contrast, a hemostatic non-risk (hemostatic stability) for said subject is determined if said concentration value determined in step (S2) is numerically closer to said at least one reference concentration value indicative for a hemostatic stability (S4'''). Steps (S3"), (S4"), and (S4''') are realized by means of the nearest neighbor approach.

The inventors had the opportunity to evaluate the risk of deep vein thrombosis by using information from the MEGA (Multiple Environment and Genetic Assessment of risk factors for venous thrombosis) study. This is a case-control study that was set up to identify risk factors for venous thrombosis that have been performed in the Netherlands. A plethora of variables, ranging from coagulation protein levels to environmental thrombotic risk factors, to age and education level, and genetic thrombophilia has been taken from patients with venous thrombosis and controls. For the purpose of the present study, the inventors used the coagulation protein levels that were measured in the MEGA study to simulate the coagulation response for a range of tissue factor levels and identified the location of the tissue factor threshold for each patient. The inventors combined these model based risk factors with direct risk factors (i.e. clinical risk factors like recent surgery, genetic data and the numerical values of the same coagulation protein levels that are used in the model) in a regression method, to arrive at one thrombosis risk score for each patient. The identified combinatory risk score is validated in an internal cross-validation on the MEGA study.

MEGA: The MEGA study (Multiple Environmental and Genetic Assessment of risk factors for venous thrombosis study) is a large, population based case-control study on risk factors for venous thrombosis, of which details have been published previously. In brief, between March 1999 and September 2004, consecutive patients aged 18 to 70 years with a first objectively confirmed episode of deep venous thrombosis or pulmonary embolism were included from six participating anticoagulation clinics in the Netherlands. Information on the diagnostic procedure was obtained from hospital records and general practitioners.

Only patients with a diagnosis of venous thrombosis that was confirmed with objective techniques were included in the analyses. Exclusion criteria were severe psychiatric problems and inability to speak Dutch. Of the 6567 eligible patients, 5184 participated (79%). For the present analysis, patients with arm vein thrombosis (n=228) and with pulmonary embolism were excluded (n=2069) to optimize the dataset with LETS in which only patients with DVT were included. As control persons, partners of patients aged <70 years without venous thrombosis were included (n=3277), as well as persons without venous thrombosis obtained via a random-digit-dialing (RDD) method (n=3000).

DATA COLLECTION: All persons were asked to complete an extensive questionnaire on many potential risk factors for venous thrombosis. Of particular interest for this study question are items on general health characteristics (age, sex, and immobilization). The index date was the date of the thrombotic event for patients and their partners, and the date of filling in the questionnaire for the random controls. The questionnaire also included questions about the presence of liver disease, kidney disease, rheumatoid arthritis, multiple sclerosis, heart failure, hemorrhagic stroke, and arterial thrombosis (myocardial infarction, angina, ischemic stroke, transient ischemic attack, and peripheral vascular disease) in the medical history.

For the current study the inventors selected the following risk factors: immobilization (plaster cast, extended bed rest at home for at least 4 days, hospitalization), surgery, a family history of venous thrombosis (considered positive if at least 1 parent, brother, or sister experienced venous thrombosis), leg injury in the past 3 months, cancer in the period from five years before to six month after the index date, travel for more than four hours in the past 2 months, pregnancy or puerperium within 3 months before the index date, or use of estrogens (oral contraceptives or hormone replacement therapy) at the index date. A further feature was the presence of obesity, determined as a body mass index of 30 kg/m$^2$ or higher.

GENETIC EFFECTS: Next to the data from the questionnaire, data was available on the presence of five genetic aspects, i.e. blood group non-O and four single nucleotide polymorphisms (SNPs) in F2 (rs1799963), fibrinogen (rs2066865), F11 (rs2036914) and F5 (FV Leiden; rs6025). The data further included the number of alleles that were affected per SNP.

BLOOD COLLECTION: Blood samples were taken at least 3 months after thrombosis was diagnosed. Whole blood (0.9 Vol.) was collected as previously described, from the antecubital vein into Sarstedt Monovette tubes (Nümbrecht, Germany) containing 0.106 M of trisodium citrate (0.1 Vol.). Plasma was prepared by centrifugation for 10 min at 2000 g at room temperature and stored in aliquots at -70 °C until assayed. All protein factor assays were previously performed and are either activity or antigen-based clinical assays. The included proteins are anti-thrombin (AT), prothrombin (factor II), factor 7 (FVII), FVIII, FIX, FX, FXI, fibrinogen and protein C (all activity measurements) and protein S (antigen measurement).

Individuals for whom no blood measurement was taken (1504 patients and 3357 controls) or less than 5 risk factors (protein levels, genetic effects and answers regarding the selected clinical risk factors) were available (n=1512 patients and n=3362) were excluded from this study. Patients that were on oral anticoagulant treatment at time of blood draw (n=294) and controls (n=34) were also excluded.

The final selection included 1227 patients and 2905 controls.

MODEL BASED RISK FACTORS - THE SENSITIVITY TO A CLOTTING TRIGGER INDICATES THROMBOSIS RISK: It is hypothesized that patients at increased risk of thrombosis start clotting at milder clotting triggers. The inventors hypothesize first that there is a threshold effect for the size of the clotting trigger. Taking the tissue factor trigger as an example, it is clear that no clotting should occur in the absence of tissue factor (no breach in the vessel wall). Very small yet non-zero tissue factor concentrations, corresponding to micro breaches of the vessel wall that occur regularly, should not lead to full blown coagulation. Serious breaches, corresponding to larger concentrations of tissue factor should however start a strong coagulation response, so somewhere between the two there should be a tissue factor threshold concentration where little to no coagulation changes into strong coagulation. The tissue factor concentration that corresponds to this threshold is taken as an indicative feature for thrombosis risk.

The coagulation response is calculated for a range of TF concentrations between 0.00004 fM and 40 nM. The range starts at an extremely low value to be sure to catch the first threshold; this threshold typically lies at TF concentrations higher than 0.4 fM and lower than 0.4 nM.

An example of the threshold effect is shown in Figure 1, where the maximum concentration (or peak height (PH)) of FVa-FXa (prothrombinase) is plotted against the size of the simulated clotting trigger. The threshold concentration is indicated as that concentration where the selected feature (in this case FVa-FXa) starts to show a strong increase. Other features may be selected as well, like the time at which the maximum occurs (time to peak (TTP)), time until a protein concentration reaches 5% of its maximum (lag time), maximum rate of change in the concentration of a protein (max rate) or the area under the plotted curve (AUC). All of these features may be calculated for various proteins that play a role in coagulation. Table 1 shows a list of such features that are used to obtain the results that are described under RESULTS.

NEAREST NEIGHBOR APPROACH: To estimate whether a patient is at high or low risk according to one feature (e.g. the tissue factor threshold determined on the model output of maximum FVa-FXa concentration), it is to be proceeded as follows:

> Calculate the feature for N subjects for whom the label (thrombosis/no thrombosis) is known
> Calculate the same feature for the new patient
> Collect the labels from the K subjects that have a predicted feature value that is numerically closest to the predicted feature for the new patient
> Of the K collected labels, $n_{case}$ will indicate thrombosis and $n_{control}$ will not. The risk score for the new patient will now be calculated as $[n_{case}/K]/Z$, where Z is the fraction of thrombosis patients in the N subjects from step 1.

COMBINING BIOMARKERS: The next step is to combine the newly created biomarker features together, and with the earlier biomarker features: clinical risk factors, SNPs and protein concentrations. This rather large set of possibly predictive features is used as the input for data driven classification methods like neural networks. In the following the inventors applied a logistic regression based approach to infer the optimal risk score from the MEGA data. The scoring method is tested internally through a 500-fold cross-validation. Here, the inventors divided the 3866 participants randomly into a training set of 2577 participants and a non-overlapping test set of 1289 participants, under the constraint that both sets have the same ratio of cases to controls. The information in the training set, i.e. the aforementioned clinical risk factors, SNPs and protein levels for each subject, and a label (1/0) to indicate whether deep vein thrombosis (DVT) was diagnosed in a subject, is used to infer a risk score. This risk score is a numerical algorithm that takes numerical values for the patient features as inputs and produces a risk number between zero and one as output.

The inferred algorithm is subsequently used to calculate a risk number for each patient in the test set. The risk scores, along with the true subject case/control labels, are used to plot a ROC curve and the area under the curve (AUC) is a measure of the methods accuracy. This process is repeated 500 times, each time with a different random split of the data into training and test set. The average AUC and 95% confidence interval are presented to evaluate the method.

ROC CURVE: the proposed method assigns a risk score between zero and one for each subject in the study. If this score is compared to a number (threshold) between zero and one and thrombosis is predicted for those with a score above that threshold and no thrombosis for those below, a sensitivity (percentage of thrombosis patients for whom thrombosis is (correctly) predicted) and a specificity (1 minus the percentage of subjects without thrombosis for whom thrombosis is (erroneously) predicted) can be calculated. Depending on the thresholds, a range of combinations from 0% sensitivity with 100% specificity to 100% sensitivity with 0% specificity can be obtained. The ROC curve plots sensitivity on the y-axis and 1-specificity on the x-axis. The area under the curve (AUC) is often used as a quality measure of a risk score.

RESULTS: For calculation of the clotting trigger threshold the inventors considered the model output features as indicated in the left column of Table 1. The univariate AUCs (based on the ROC curve obtained using the related tissue factor threshold as the sole element in the risk score) given in the second column. Threshold features that scored 0.65 or more were found for total thrombin production (maximum rate), most FVIIIa-FIXa features, TFPI peak height, ETP and FII-FXa peak height.

Table 1: Model output features for the calculation of the clotting trigger.

| | |
|---|---|
| Total thrombin production | 0.66 |
| Maximum rate of total thrombin | 0.65 |
| FVa-FXa PH | 0.61 |
| FVa-FXa TTP | 0.61 |
| FVa-FXa lag | 0.58 |
| FVa-FXa max rate | 0.63 |
| FVa-FXa AUC | 0.62 |
| FVIIIa-FIXa PH | 0.67 |
| FVIIIa-FIXa TTP | 0.67 |
| FVIIIa-FIXa lag | 0.64 |
| FVIIIa-FIXa max rate | 0.67 |
| FVIIIa-FIXa AUC | 0.67 |
| TFPI PH | 0.66 |
| TFPI max rate | 0.63 |
| FIIa PH | 0.64 |
| FIIa TTP | 0.64 |
| FIIa lag | 0.61 |
| FIIa max rate | 0.63 |
| FIIa AUC | 0.65 |
| FVIII-FIIa PH | 0.59 |
| FVIII-FIIa TTP | 0.59 |
| FVIII-FIIa lag | 0.58 |
| FVIII-FIIa max rate | 0.60 |

(continued)

| | |
|---|---|
| FVIII-FIIa AUC | 0.61 |
| FII-FXa PH | 0.68 |
| FII-FXa TTP | 0.63 |
| FII-FXa max rate | 0.63 |
| Fibrin TTP | 0.61 |
| Fibrin lag | 0.58 |

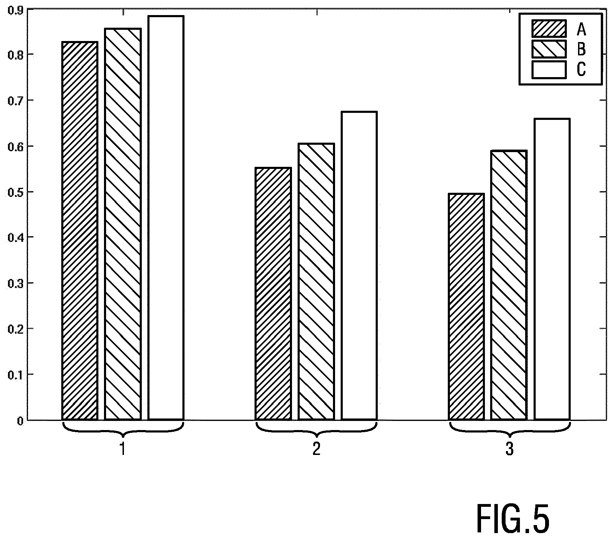

REGRESSION ON DIRECT FEATURES AND CLOTTING TRIGGER THRESHOLDS: Here a standard logistic regression function was used to evaluate the risk score based on clinical risk factors, SNPs, protein levels and model based thresholds in a cross validation study on the MEGA database. For the cross-validation the inventors make 500 random divisions of the data into a train and a validation set (2:1), where it was made sure that the case-control ratio is the same in both sets. For the threshold the inventors used a nearest neighbor score as described above, where all neighbors must be in the training set. The result is shown in Fig. 5 (A: CRF+SNP; B: CRF+SNP+Proteins; C: CRF+SNP+Proteins+Thresholds). The area under the ROC curve (Fig. 5, set of three bars on the left (1)), and correspondingly the maximum sensitivity that can be obtained in combination with 90% specificity (Fig. 5, set of three bars in the middle (2)) (and vice versa, the maximum specificity that can be obtained in combination with 90% sensitivity (Fig. 5, set of three bars on the right (3)) are significantly improved when tissue factor threshold features are included in the risk score. When the inventors compared the improvement due to inclusion of measured protein concentrations (from the bars to the very left to the bars in the middle of each set) to the total improvement (from the bars in the middle to the bars to the very right of each set), it can be seen that inclusion of the calculated tissue factor threshold, based on the exact same measured protein concentrations (i.e. requiring no additional measurement), doubles the increase in risk estimation accuracy.

SELECTION OF THE MOST INFORMATIVE FEATURES: The inventors performed a pruning method to identify the most relevant features. They proceeded as follows:

Making 500 divisions into 1/3 validation set and 2/3 train set
On the train set taking 100 random divisions into 1/3 test set and 2/3 (remains) train set
At each iteration removing, one by one, the feature that leads to the smallest decrease in estimation accuracy (AUC) on the training set. If multiple features cause the same (minimum) decrease in accuracy, pick one of these at random.

Store the number of times that each feature survives the pruning step described in the previous item, and the drop in accuracy caused by the removal of this feature.

After 100 iterations rank the features, first by number of pruning steps survived, second by average drop in accuracy upon removal.

Remove the features one by one in order of their ranking, and calculate the AUC on the 100 test sets.

For each of the 500 divisions, identify the minimum number of features that still leads to an average AUC on the test sets that exceeds the maximum average score on the test set minus one standard deviation (calculated around the maximum average score on the test set).

Count the number of times that each feature ends up in the remaining minimum feature set as described in the previous bullet point, over the 500 iterations.

On average 25 features end up in the selection made in the penultimate bullet point (95%CI=[19,31]). The ranking of the features that remain most often is shown in Table 2. It can be seen that the features that were most important in the previously published data driven approach (FVIII, FVLeiden, leg injury, etc.) still score high, but are now augmented in the top ten by two calculated threshold based features, i.e. the thresholds in total activated thrombin and endogenous thrombin potential.

Table 2: Ranking of the features most indicative for a hemostatic risk

| Feature name | Percentage selected | Feature name | Percentage selected |
|---|---|---|---|
| FVIII level | 100 | Pregnancy | 100 |
| FV Leiden SNP | 100 | Gender | 100 |

(continued)

| Feature name | Percentage selected | Feature name | Percentage selected |
|---|---|---|---|
| Leg injury | 100 | Plaster cast | 100 |
| Recent operation | 100 | Malignancies | 99 |
| Family history | 100 | FII SNP | 89 |
| Oral contraceptive use | 100 | TF threshold on FVIIIa-FIXa max generation rate | 86 |
| Immobility (hospital) | 100 | TF threshold on FVa-FXa Peak Height | 63 |
| Immobility (home) | 100 | TF threshold on maximum FVIIIa-FIXa | 62 |
| Free Protein S level | 100 | TF threshold on FVa-FXa max generation rate | 60 |
| Fibrinogen SNP | 100 | Obesity | 58 |
| TF threshold on tot. FIIa | 100 | TF threshold on maximum TFPI | 52 |
| TF threshold on ETP | 100 | Blood group | 52 |

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method for determining a hemostatic risk of a subject, the method comprising a comparison of a concentration value of a clotting trigger sufficient to start the clotting process in a subject with at least one reference concentration value of said clotting trigger indicative for a hemostatic risk and/or a hemostatic non-risk (hemostatic stability), said method comprising the steps of:

a) providing a first information on the hemostatic condition of said subject (S1), wherein said first information is the concentration values of a plurality of coagulation proteins in a blood sample from said subject,

b) determining on the basis of said first information of step (a) a concentration value of a clotting trigger sufficient to start the clotting process in said subject (S2), wherein said clotting trigger is tissue factor, TF, wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,

c) comparing said concentration value determined in step (b) with a reference concentration value of said TF representing in healthy reference subjects the minimum concentration for a stable hemostatic condition (S3), and

d) determining a high risk of thrombosis for said subject if said concentration value determined in step (b) is lower than said reference concentration value of said TF representing in healthy reference subjects the minimum concentration for a stable hemostatic condition (S4).

2. The method of claim 1, wherein steps c) and d), additionally comprise the following steps:

    c') comparing said concentration value determined in step (b) with a reference concentration value of said TF representing in healthy reference subjects the maximum concentration for a stable hemostatic condition (S3'), and
    d') determining a high risk of bleeding for said subject if said concentration value determined in step (b) is higher than said reference concentration value of said TF representing in healthy reference subjects the maximum concentration for a stable hemostatic condition (S4').

3. A computer-implemented method for determining a hemostatic risk of a subject, the method comprising a comparison of a concentration value of a clotting trigger sufficient to start the clotting process in a subject with at least one reference concentration value of said clotting trigger indicative for a hemostatic risk and/or a hemostatic non-risk (hemostatic stability), said method comprising the steps of:

    a) providing a first information on the hemostatic condition of said subject (S1), wherein said first information is the concentration values of a plurality of coagulation proteins in a blood sample from said subject,
    b) determining on the basis of said first information of step (a) a concentration value of a clotting trigger sufficient to start the clotting process in said subject (S2), wherein said clotting trigger is TF, wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,
    c) comparing said concentration value determined in step (b) with at least two or more reference concentration values of said TF comprising at least one reference concentration value indicative in healthy reference subjects for a hemostatic risk, preferably a high risk of thrombosis and/or a high risk of bleeding, and at least one reference concentration value indicative in healthy reference subjects for a hemostatic non-risk (hemostatic stability) (S3"), and
    d) determining

        - a hemostatic risk for said subject if said concentration value determined in step (b) is numerically closer to said at least one reference concentration value indicative in healthy reference subjects for a hemostatic risk (S4"), or
        - a hemostatic non-risk (hemostatic stability) for said subject if said concentration value determined in step (b) is numerically closer to said at least one reference concentration value indicative in healthy subjects for a hemostatic stability (S4'''),
    wherein preferably steps (c) and (d) are realized by means of a nearest neighbor approach.

4. The method of claim 1, 2 or 3, wherein in step (a) said first information on the hemostatic condition of said subject is the concentration values of at least three or more coagulation proteins in a biological sample from said subject, wherein further preferably said coagulation protein(s) is/are selected from the group consisting of: coagulation factor 2 (FII), FV, FVII, FVIII, FIX, FX, FXI, FXII, antithrombin (AT), TFPI, $\alpha$2M, C4BP, protein C, protein S, protein Z, TAFI, ZPI, AAT, PCI, C1 inhibitor and fibrinogen.

5. The method of claim 1, wherein said second information on the hemostatic condition of said subject is the concentration values of a plurality of activated coagulation proteins at a series of time points of the simulated clotting process which are used as a set of output features, wherein most preferably said activated coagulation protein(s) is/are selected from the group consisting of: thrombin (FIIa), FVa, FVIIa, FVIIIa, FIXa, FXa, FXIIa, FVa-FXa, FVIIIa-FIXa, fibrin, prothrombin (FII).

6. The method of claim 5, wherein out of the set of output features one feature is created representing the strength of the clotting response, wherein preferably said one feature representing the strength of the clotting response is the maximum concentration of at least one of said activated coagulation proteins over all time points of the simulated clotting process.

7. A device for determining a hemostatic risk of a subject, said device comprising:

a receiving unit configured for receiving a first information on the hemostatic condition of said subject, wherein said first information is the concentration values of a plurality of coagulation proteins in a blood sample from said subject,
a first determining unit configured for determining on the basis of said first information received by the receiving unit a concentration value of a clotting trigger sufficient to start the clotting process in said subject, wherein said clotting trigger is tissue factor, TF, wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF, and
a comparing unit configured for comparing said concentration value determined by said first determining unit with at least one reference concentration value of said TF indicative in healthy reference subjects for a hemostatic risk and/or a hemostatic non-risk (hemostatic stability), wherein said comparing unit is configured for comparing said concentration value determined by said first determining unit with a reference concentration value of said TF representing in healthy reference subjects the minimum concentration for a stable hemostatic condition, and said device is further comprising:

a second determining unit configured for determining a high risk of thrombosis for said subject if said concentration value determined by said first determining unit is lower than said reference concentration value of said TF representing in healthy reference subjects the minimum concentration for a stable hemostatic condition;
wherein preferably, additionally or alternatively,
said comparing unit is configured for comparing said concentration value determined by said first determining unit with a reference concentration value of said TF representing in healthy reference subjects the maximum concentration for a stable hemostatic condition, and
said second determining unit is configured for determining a high risk of bleeding for said subject if said concentration value determined by said first determining unit is higher than said reference concentration value of said TF representing in healthy reference subjects the maximum concentration for a stable hemostatic condition.

8. The device of claim 7, wherein said comparing unit is configured for comparing said concentration value determined by said first determining unit with at least two or more reference concentration values of said TF comprising at least one reference concentration value indicative in healthy reference subjects for a hemostatic risk, preferably a high risk of thrombosis and/or a high risk of bleeding, and at least one reference concentration value indicative in healthy reference subjects for a hemostatic non-risk (hemostatic stability), and said device is further comprising:
a second determining unit configured for determining:

- a hemostatic risk for said subject if said concentration value determined by said first determining unit is numerically closer to said at least one reference concentration value indicative in healthy reference subjects for a hemostatic risk, or
- a hemostatic non-risk (hemostatic stability) for said subject if said concentration value determined by said first determining unit is numerically closer to said at least one reference concentration value indicative in healthy reference subjects for a hemostatic stability.

9. A clinical decision support system comprising a processor and a computer-readable storage medium, wherein said computer-readable storage medium contains instructions for execution by the processor, wherein said instructions cause said processor to perform the steps of:

a) receiving a first information on the hemostatic condition of a subject, wherein said first information is the concentration values of a plurality of coagulation proteins in a blood sample from said subject,
b) determining on the basis of said information of step (a) a concentration value of a clotting trigger sufficient to start the clotting process in said subject, wherein said clotting trigger is tissue factor, TF, wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,

c) comparing said concentration value determined in step (b) with at least one reference concentration value of said TF indicative in healthy reference subjects for a hemostatic risk and/or a hemostatic non-risk (hemostatic stability),

10. A clinical decision support system comprising a processor and a computer-readable storage medium, wherein said computer-readable storage medium contains instructions for execution by the processor, wherein said instructions cause said processor to perform the steps of:

a) providing a first information on the hemostatic condition of said subject wherein said first information is a concentration value of a plurality of coagulation proteins in a blood sample from said subject,

b) determining on the basis of said first information of step (a) a concentration value of a clotting trigger sufficient to start the clotting process in said subject wherein said clotting trigger is TF, wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,

c) comparing said concentration value determined in step (b) with a reference concentration value of said TF representing in healthy reference subjects the minimum concentration for a stable hemostatic condition, and representing in healthy reference subjects the minimum concentration for a stable hemostatic condition, and

d) determining a high risk of thrombosis for said subject if said concentration value determined in step (b) is lower than said reference concentration value of said TF representing in healthy reference subjects the minimum concentration for a stable hemostatic condition.

11. The clinical decision support system of claim 9 or 10, wherein steps c) and d) additionally comprise the following steps:

c') comparing said concentration value determined in step (b) with a reference concentration value of said TF representing in healthy reference subjects the maximum concentration for a stable hemostatic condition, and

d') determining a high risk of bleeding for said subject if said concentration value determined in step (b) is higher than said reference concentration value of said TF representing in healthy reference subjects the maximum concentration for a stable hemostatic condition.

12. A clinical decision support system comprising a processor and a computer-readable storage medium, wherein said computer-readable storage medium contains instructions for execution by the processor, wherein said instructions cause said processor to perform the steps of:

a) providing a first information on the hemostatic condition of said subject, wherein said first information is a concentration value of a plurality of coagulation proteins in a blood sample from said subject,

b) determining on the basis of said first information of step (a) a concentration value of a clotting trigger sufficient to start the clotting process in said subject, wherein said clotting trigger is TF, wherein said concentration value of TF sufficient to start the clotting process in the subject is determined by an *in silico* simulation of the clotting process, in the *in silico* simulation said first information on the hemostatic condition of said subject is used as input feature, and a second information on the hemostatic condition of said subject in the simulated clotting process is used as output feature, wherein said second information on the hemostatic condition of said subject is the concentration values of an activated coagulation protein at a series of time points of the simulated clotting process which are used as a set of output features, and wherein the *in silico* simulation simulates the dynamic change in coagulation protein concentrations after exposure to TF,

c) comparing said concentration value determined in step (b) with at least two or more reference concentration values of said TF comprising at least one reference concentration value indicative in healthy reference subjects for a hemostatic risk, preferably a high risk of thrombosis and/or a high risk of bleeding, and at least one reference concentration value indicative in healthy reference subjects for a hemostatic non-risk (hemostatic stability), and

d) determining:

- a hemostatic risk for said subject if said concentration value determined in step (b) is numerically closer to said at least one reference concentration value indicative in healthy reference subjects for a hemostatic risk, or

- a hemostatic non-risk (hemostatic stability) for said subject if said concentration value determined in step (b) is numerically closer to said at least one reference concentration value indicative in healthy reference subjects for a hemostatic stability.

13. Computer program comprising program code means for causing a computer to carry out the steps of the method of claims 1 to 6 when said computer program is carried out on the computer.

14. A computer-readable non-transitory storage medium containing instructions for execution by a processor, wherein the instructions cause the processor to perform the steps of the method of claims 1 to 6.

**Patentansprüche**

1. Computer-implementiertes Verfahren zur Bestimmung des hämostatischen Risikos einer Person, wobei das Verfahren einen Vergleich eines Konzentrationswerts eines Gerinnungsauslösers, der ausreicht, um den Gerinnungsprozess in einer Person zu starten, mit mindestens einem Referenzkonzentrationswert des genannten Gerinnungsauslösers, der auf ein hämostatisches Risiko und/oder ein hämostatisches Nicht-Risiko (hämostatische Stabilität) hinweist, umfasst, wobei das genannte Verfahren die folgenden Schritte umfasst:

a) Bereitstellen von ersten Informationen zu dem hämostatischen Zustand der genannten Person (S1), wobei die genannten ersten Informationen die Konzentrationswerte einer Vielzahl von Koagulationsproteinen in einer Blutprobe der genannten Person sind,

b) Bestimmen, ausgehend von den genannten ersten Informationen aus Schritt (a), eines Konzentrationswerts eines Gerinnungsauslösers, der ausreicht, um den Gerinnungsprozess in der genannten Person zu starten (S2), wobei der genannte Gerinnungsauslöser Gewebefaktor TF ist, wobei der genannte Konzentrationswert von TF, der ausreicht, um den Gerinnungsprozess in der Person zu starten, durch eine *in-silico*-Simulation des Gerinnungsprozesses bestimmt wird, wobei in der *in-silico*-Simulation die genannten ersten Informationen zu dem hämostatischen Zustand der genannten Person als Eingabemerkmal verwendet werden, und zweite Informationen zu dem hämostatischen Zustand der genannten Person in dem simulierten Gerinnungsprozess als Ausgabemerkmal verwendet werden, wobei die genannten zweiten Informationen zu dem hämostatischen Zustand der genannten Person die Konzentrationswerte eines aktivierten Koagulationsproteins zu einer Reihe von Zeitpunkten des simulierten Gerinnungsprozesses sind, die als ein Satz von Ausgabemerkmalen verwendet werden, und wobei die *in-silico*-Simulation die dynamische Veränderung der Koagulationsproteinkonzentrationen nach Exposition gegenüber TF simuliert,

c) Vergleichen des genannten Konzentrationswerts, der in Schritt (b) bestimmt wird, mit einem Referenzkonzentrationswert des genannten TF, der in gesunden Referenzpersonen die Mindestkonzentration für einen stabilen hämostatischen Zustand darstellt (S3), und

d) Bestimmen eines hohen Thromboserisikos für die genannte Person, wenn der genannte Konzentrationswert, der in Schritt (b) bestimmt wird, niedriger ist als der genannte Referenzkonzentrationswert des genannten TF, der in gesunden Referenzpersonen die Mindestkonzentration für einen stabilen hämostatischen Zustand darstellt (S4).

2. Verfahren nach Anspruch 1, wobei die Schritte c) und d) zusätzlich die folgenden Schritte umfassen:

c') Vergleichen des genannten Konzentrationswerts, der in Schritt (b) bestimmt wird, mit einem Referenzkonzentrationswert des genannten TF, der in gesunden Referenzpersonen die maximale Konzentration für einen stabilen hämostatischen Zustand darstellt (S3'), und

d') Bestimmen eines hohen Blutungsrisikos für die genannte Person, wenn der genannte Konzentrationswert, der in Schritt (b) bestimmt wird, höher ist als der genannte Referenzkonzentrationswert des genannten TF, der in gesunden Referenzpersonen die maximale Konzentration für einen stabile hämostatischen Zustand darstellt (S4').

3. Computer-implementiertes Verfahren zur Bestimmung des hämostatischen Risikos einer Person, wobei das Verfahren einen Vergleich eines Konzentrationswerts eines Gerinnungsauslösers, der ausreicht, um den Gerinnungsprozess in einer Person zu starten, mit mindestens einem Referenzkonzentrationswert des genannten Gerinnungsauslösers, der auf ein hämostatisches Risiko und/oder ein hämostatisches Nicht-Risiko (hämostatische Stabilität) hinweist, umfasst, wobei das genannte Verfahren die folgenden Schritte umfasst:

a) Bereitstellen von ersten Informationen zu dem hämostatischen Zustand der genannten Person (S1), wobei die genannten ersten Informationen die Konzentrationswerte einer Vielzahl von Koagulationsproteinen in einer Blutprobe der genannten Person sind,

b) Bestimmen, ausgehend von den genannten ersten Informationen aus Schritt (a), eines Konzentrationswerts eines Gerinnungsauslösers, der ausreicht, um den Gerinnungsprozess in der genannten Person zu starten (S2), wobei der genannte Gerinnungsauslöser TF ist, wobei der genannte Konzentrationswert von TF, der ausreicht, um den Gerinnungsprozess in der Person zu starten, durch eine in silico Simulation des Gerinnungsprozesses bestimmt wird, wobei in der *in-silico*-Simulation die genannten ersten Informationen zu dem hämostatischen Zustand der genannten Person als Eingabemerkmal verwendet werden, und zweite Informationen zu dem hämostatischen Zustand der genannten Person in dem simulierten Gerinnungsprozess als Ausgabemerkmal verwendet werden, wobei die genannten zweiten Informationen zu dem hämostatischen Zustand der genannten Person die Konzentrationswerte eines aktivierten Koagulationsproteins zu einer Reihe von Zeitpunkten des simulierten Gerinnungsprozesses sind, die als ein Satz von Ausgabemerkmalen verwendet werden, und wobei die *in-silico*-Simulation die dynamische Veränderung der Koagulationsproteinkonzentrationen nach Exposition gegenüber TF simuliert,

c) Vergleichen des genannten Konzentrationswerts, der in Schritt (b) bestimmt wird, mit mindestens zwei oder mehr Referenzkonzentrationswerten des genannten TF, die mindestens einen Referenzkonzentrationswert, welcher in gesunden Referenzpersonen auf ein hämostatisches Risiko hinweist, vorzugsweise auf ein hohes Thromboserisiko und/oder ein hohes Blutungsrisiko, und mindestens einen Referenzkonzentrationswert, der in gesunden Referenzpersonen auf ein hämostatisches Nicht-Risiko (hämostatische Stabilität) hinweist, umfassen (S3"), und

d) Bestimmen

- eines hämostatischen Risikos für die genannte Person, wenn der genannte Konzentrationswert, der in Schritt (b) bestimmt wird, numerisch näher an dem genannten mindestens einen Referenzkonzentrationswert liegt, der in gesunden Referenzpersonen auf ein hämostatisches Risiko hinweist (S4"), oder
- eines hämostatischen Nicht-Risikos (hämostatische Stabilität) für die genannte Person, wenn der genannte Konzentrationswert, der in Schritt (b) bestimmt wird, numerisch näher an dem genannten mindestens einen Referenzkonzentrationswert liegt, der in gesunden Personen auf eine hämostatische Stabilität hinweist (S4'''),
wobei vorzugsweise die Schritte (c) und (d) mittels einer Nächster-Nachbar-Vorgehensweise realisiert werden.

4. Verfahren nach Anspruch 1, 2 oder 3, wobei in Schritt (a) die genannten ersten Informationen zu dem hämostatischen Zustand der genannten Person die Konzentrationswerte von mindestens drei oder mehr Koagulationsproteinen in einer biologischen Probe von der genannten Person sind, wobei ferner das/die genannte(n) Koagulationsprotein(e) vorzugsweise ausgewählt ist/sind aus der Gruppe bestehend aus: Koagulationsfaktor 2 (FII), FV, FVII, FVIII, FIX, FX, FXI, FXII, Antithrombin (AT), TFPI, α2M, C4BP, Protein C, Protein S, Protein Z, TAFI, ZPI, AAT, PCI, C1-Hemmer und Fibrinogen.

5. Verfahren nach Anspruch 1, wobei die genannten zweiten Informationen zu dem hämostatischen Zustand der genannten Person die Konzentrationswerte einer Vielzahl von aktivierten Koagulationsproteinen zu einer Reihe von Zeitpunkten des simulierten Gerinnungsprozesses sind, die als ein Satz von Ausgabemerkmalen verwendet werden, wobei das/die genannte(n) aktivierte(n) Koagulationsprotein€ am meisten bevorzugt ausgewählt wird/werden aus der Gruppe bestehend aus: Thrombin (FIIa), FVa, FVIIa, FVIIIa, FIXa, FXa, FXIIa, FVa-FXa, FVIIIa-FIXa, Fibrin, Prothrombin (FII).

6. Verfahren nach Anspruch 5, wobei ein Merkmal aus dem Satz von Ausgabemerkmalen erstellt wird, das die Stärke der Gerinnungsantwort darstellt, wobei das genannte eine Merkmal, das die Stärke der Gerinnungsantwort darstellt, vorzugsweise die maximale Konzentration von mindestens einem der genannten aktivierten Koagulationsproteine über alle Zeitpunkte des simulierten Gerinnungsprozesses ist.

7. Vorrichtung zur Bestimmung des hämostatischen Risikos einer Person, wobei die genannte Vorrichtung Folgendes umfasst:

eine Empfangseinheit, die konfiguriert ist, um erste Informationen zu dem hämostatischen Zustand der genannten Person zu empfangen, wobei die genannten ersten Informationen die Konzentrationswerte einer Vielzahl von Koagulationsproteinen in einer Blutprobe der genannten Person sind,
eine erste Bestimmungseinheit, die konfiguriert ist, um ausgehend von den durch die Empfangseinheit empfangenen ersten Informationen einen Konzentrationswert eines Gerinnungsauslösers zu bestimmen, der ausreicht, um den Gerinnungsprozess in der genannten Person zu starten, wobei der genannte Gerinnungsauslöser Gewebefaktor TF ist, wobei der genannte Konzentrationswert von TF, der ausreicht, um den Gerinnungsprozess in der Person zu starten, durch eine *in-silico*-Simulation des Gerinnungsprozesses bestimmt wird, wobei in der *in-silico*-Simulation die genannten ersten Informationen zu dem hämostatischen Zustand der genannten Person als Eingabemerkmal verwendet werden, und zweite Informationen zu dem hämostatischen Zustand der genannten Person in dem simulierten Gerinnungsprozess als Ausgabemerkmal verwendet werden, wobei die genannten zweiten Information zu dem hämostatischen Zustand der genannten Person die Konzentrationswerte eines aktivierten Koagulationsproteins zu einer Reihe von Zeitpunkten des simulierten Gerinnungsprozesses sind, die als ein Satz von Ausgabemerkmalen verwendet werden, und wobei die *in-silico*-Simulation die dynamische Veränderung der Koagulationsproteinkonzentrationen nach Exposition gegenüber TF simuliert, und
eine Vergleichseinheit, die konfiguriert ist, um den genannten Konzentrationswert, der durch die genannte Bestimmungseinheit bestimmt wird, mit einem Referenzkonzentrationswert des genannten TF zu vergleichen, der in gesunden Referenzpersonen auf ein hämostatisches Risiko und/oder ein hämostatisches Nicht-Risiko (hämostatische Stabilität) hinweist, wobei die genannte Vergleichseinheit konfiguriert ist, um den genannten Konzentrationswert, der durch die genannte erste Bestimmungseinheit bestimmt wird, mit einem Referenzkonzentrationswert des genannten TF zu vergleichen, der in gesunden Referenzpersonen die Mindestkonzentration für einen stabilen hämostatischen Zustand darstellt, und wobei die genannte Vorrichtung ferner Folgendes umfasst:

eine zweite Bestimmungseinheit, die konfiguriert ist, um ein hohes Thromboserisiko für die genannte Person zu bestimmen, wenn der genannte Konzentrationswert, der durch die genannte erste Bestimmungseinheit bestimmt wird, niedriger ist als der genannte Referenzkonzentrationswert des genannten TF, der in gesunden Referenzpersonen die Mindestkonzentration für einen stabilen hämostatischen Zustand darstellt;.
wobei vorzugsweise, zusätzlich oder alternativ
die genannte Vergleichseinheit konfiguriert ist, um den genannten Konzentrationswert, der durch die genannte erste Bestimmungseinheit bestimmt wird, mit einem Referenzkonzentrationswert des genannten TF zu vergleichen, der in gesunden Referenzpersonen die maximale Konzentration für einen stabilen hämostatischen Zustand darstellt, und
die genannte zweite Bestimmungseinheit konfiguriert ist, um ein hohes Blutungsrisiko für die genannte Person zu bestimmen, wenn der genannte Konzentrationswert, der durch die erste Bestimmungseinheit bestimmt wird, höher ist als der genannte Referenzkonzentrationswert des genannten TF, der in gesunden Referenzpersonen die maximale Konzentration für einen stabilen hämostatischen Zustand darstellt.

8. Vorrichtung nach Anspruch 7, wobei die genannte Vergleichseinheit konfiguriert ist, um den genannten Konzentrationswert, der durch die genannte erste Bestimmungseinheit bestimmt wird, mit mindestens zwei oder mehr Referenzkonzentrationswerten des genannten TF zu vergleichen, die mindestens einen Referenzkonzentrationswert, welcher in gesunden Referenzpersonen auf ein hämostatisches Risiko hinweist, vorzugsweise auf ein hohes Thromboserisiko und/oder ein hohes Blutungsrisiko, und mindestens einen Referenzkonzentrationswert, der in gesunden Referenzpersonen auf ein hämostatisches Nicht-Risiko (hämostatische Stabilität) hinweist, umfassen, und wobei die genannte Vorrichtung ferner Folgendes umfasst:
eine zweite Bestimmungseinheit, die konfiguriert ist zum Bestimmen

- eines hämostatischen Risikos für die genannte Person, wenn der genannte Konzentrationswert, der durch die erste Bestimmungseinheit bestimmt wird, numerisch näher an dem genannten mindestens einen Referenzkonzentrationswert liegt, der in gesunden Referenzpersonen auf ein hämostatisches Risiko hinweist, oder
- eines hämostatischen Nicht-Risikos (hämostatische Stabilität) für die genannte Person, wenn der genannte Konzentrationswert, der durch die genannte erste Bestimmungseinheit bestimmt wird, numerisch näher an dem genannten mindestens einen Referenzkonzentrationswert liegt, der in gesunden Referenzpersonen auf eine hämostatische Stabilität hinweist.

9. Klinisches Entscheidungsunterstützungssystem umfassend einen Prozessor und ein computerlesbares Speichermedium, wobei das genannte computerlesbare Speichermedium Anweisungen zur Ausführung durch einen Prozessor enthält, wobei die genannten Anweisungen den genannten Prozessor veranlassen, die folgenden Schritte durchzuführen:

    a) Empfangen von ersten Informationen zu dem hämostatischen Zustand einer Person, wobei die genannten ersten Informationen die Konzentrationswerte einer Vielzahl von Koagulationsproteinen in einer Blutprobe der genannten Person sind,

    b) Bestimmen, ausgehend von genannten Informationen aus Schritt (a), eines Konzentrationswerts eines Gerinnungsauslösers, der ausreicht, um den Gerinnungsprozess in der genannten Person zu starten, wobei der genannte Gerinnungsauslöser Gewebefaktor TF ist, wobei der genannte Konzentrationswert von TF, der ausreicht, um den Gerinnungsprozess in der Person zu starten, durch eine *in-silico*-Simulation des Gerinnungsprozesses bestimmt wird, wobei in der *in-silico*-Simulation die genannten ersten Informationen zu dem hämostatischen Zustand der genannten Person als Eingabemerkmal verwendet werden, und zweite Informationen zu dem hämostatischen Zustand der genannten Person in dem simulierten Gerinnungsprozess als Ausgabemerkmal verwendet werden, wobei die genannten zweiten Information zu dem hämostatischen Zustand der genannten Person die Konzentrationswerte eines aktivierten Koagulationsproteins zu einer Reihe von Zeitpunkten des simulierten Gerinnungsprozesses sind, die als ein Satz von Ausgabemerkmalen verwendet werden, und wobei die *in-silico*-Simulation die dynamische Veränderung der Koagulationsproteinkonzentrationen nach Exposition gegenüber TF simuliert,

    c) Vergleichen des genannten Konzentrationswerts, der in Schritt (b) bestimmt wird, mit mindestens einem Referenzkonzentrationswert des genannten TF, der in gesunden Referenzpersonen auf ein hämostatisches Risiko und/oder ein hämostatisches Nicht-Risiko (hämostatische Stabilität) hinweist.

10. Klinisches Entscheidungsunterstützungssystem umfassend einen Prozessor und ein computerlesbares Speichermedium, wobei das genannte computerlesbare Speichermedium Anweisungen zur Ausführung durch einen Prozessor enthält, wobei die genannten Anweisungen den genannten Prozessor veranlassen, die folgenden Schritte durchzuführen:

    a) Bereitstellen ersten Informationen zu dem hämostatischen Zustand der genannten Person, wobei die genannten ersten Informationen ein Konzentrationswert einer Vielzahl von Koagulationsproteinen in einer Blutprobe der genannten Person sind,

    b) Bestimmen, ausgehend von den genannten ersten Informationen aus Schritt (a), eines Konzentrationswerts eines Gerinnungsauslösers, der ausreicht, um den Gerinnungsprozess in der genannten Person zu starten, wobei der genannte Gerinnungsauslöser TF ist, wobei der genannte Konzentrationswert von TF, der ausreicht, um den Gerinnungsprozess in der Person zu starten, durch eine *in-silico*-Simulation des Gerinnungsprozesses bestimmt wird, wobei in der *in-silico*-Simulation die genannten ersten Informationen zu dem hämostatischen Zustand der genannten Person als Eingabemerkmal verwendet werden, und zweite Informationen zu dem hämostatischen Zustand der genannten Person in dem simulierten Gerinnungsprozess als Ausgabemerkmal verwendet werden, wobei die genannten zweiten Informationen zu dem hämostatischen Zustand der genannten Person die Konzentrationswerte eines aktivierten Koagulationsproteins zu einer Reihe von Zeitpunkten des simulierten Gerinnungsprozesses sind, die als ein Satz von Ausgabemerkmalen verwendet werden, und wobei die *in-silico*-Simulation die dynamische Veränderung der Koagulationsproteinkonzentrationen nach Exposition gegenüber TF simuliert,

    c) Vergleichen des genannten Konzentrationswerts, der in Schritt (b) bestimmt wird, mit einem Referenzkonzentrationswert des genannten TF, der in gesunden Referenzpersonen die Mindestkonzentration für einen stabilen hämostatischen Zustand darstellt, und der in gesunden Referenzpersonen die Mindestkonzentration für einen stabilen hämostatischen Zustand darstellt und

    d) Bestimmen eines hohen Thromboserisikos für die genannte Person, wenn der genannte Konzentrationswert, der in Schritt (b) bestimmt wird, niedriger ist als der genannte Referenzkonzentrationswert des genannten TF, der in gesunden Referenzpersonen die Mindestkonzentration für einen stabilen hämostatischen Zustand darstellt.

11. Klinisches Entscheidungsunterstützungssystem nach Anspruch 9 oder 10, wobei die Schritte c) und d) zusätzlich die folgenden Schritte umfassen:

    c') Vergleichen des genannten Konzentrationswerts, der in Schritt (b) bestimmt wird, mit einem Referenzkonzentrationswert des genannten TF, der in gesunden Referenzpersonen die maximale Konzentration für einen stabilen hämostatischen Zustand darstellt, und

d') Bestimmen eines hohen Blutungsrisikos für die genannte Person, wenn der genannte Konzentrationswert, der in Schritt (b) bestimmt wird, höher ist als der genannte Referenzkonzentrationswert des genannten TF, der in gesunden Referenzpersonen die maximale Konzentration für einen stabile hämostatischen Zustand darstellt.

12. Klinisches Entscheidungsunterstützungssystem umfassend einen Prozessor und ein computerlesbares Speichermedium, wobei das genannte computerlesbare Speichermedium Anweisungen zur Ausführung durch einen Prozessor enthält, wobei die genannten Anweisungen den genannten Prozessor veranlassen, die folgenden Schritte durchzuführen:

a) Bereitstellen ersten Informationen zu dem hämostatischen Zustand der genannten Person, wobei die genannten ersten Informationen ein Konzentrationswert einer Vielzahl von Koagulationsproteinen in einer Blutprobe der genannten Person sind,

b) Bestimmen, ausgehend von der genannten ersten Information aus Schritt (a), eines Konzentrationswerts eines Gerinnungsauslösers, der ausreicht, um den Gerinnungsprozess in der genannten Person zu starten, wobei der genannte Gerinnungsauslöser TF ist, wobei der genannte Konzentrationswert von TF, der ausreicht, um den Gerinnungsprozess in der Person zu starten, durch eine *in-silico*-Simulation des Gerinnungsprozesses bestimmt wird, wobei in der *in-silico*-Simulation die genannten ersten Informationen zu dem hämostatischen Zustand der genannten Person als Eingabemerkmal verwendet werden, und zweite Informationen zu dem hämostatischen Zustand der genannten Person in dem simulierten Gerinnungsprozess als Ausgabemerkmal verwendet werden, wobei die genannten zweiten Informationen zu dem hämostatischen Zustand der genannten Person die Konzentrationswerte eines aktivierten Koagulationsproteins zu einer Reihe von Zeitpunkten des simulierten Gerinnungsprozesses sind, die als ein Satz von Ausgabemerkmalen verwendet werden, und wobei die *in-silico*-Simulation die dynamische Veränderung der Koagulationsproteinkonzentrationen nach Exposition gegenüber TF simuliert,

c) Vergleichen des genannten Konzentrationswerts, der in Schritt (b) bestimmt wird, mit mindestens zwei oder mehr Referenzkonzentrationswerten des genannten TF, die mindestens einen Referenzkonzentrationswert, welcher in gesunden Referenzpersonen auf ein hämostatisches Risiko hinweist, vorzugsweise auf ein hohes Thromboserisiko und/oder ein hohes Blutungsrisiko, und mindestens einen Referenzkonzentrationswert, der in gesunden Referenzpersonen auf ein hämostatisches Nicht-Risiko (hämostatische Stabilität) hinweist, umfassen, und

d) Bestimmen

- eines hämostatischen Risikos für die genannte Person, wenn der genannte Konzentrationswert, der in Schritt (b) bestimmt wird, numerisch näher an dem genannten mindestens einen Referenzkonzentrationswert liegt, der in gesunden Referenzpersonen auf ein hämostatisches Risiko hinweist, oder
- eines hämostatischen Nicht-Risikos (hämostatische Stabilität) für die genannte Person, wenn der genannte Konzentrationswert, der in Schritt (b) bestimmt wird, numerisch näher an dem genannten mindestens einen Referenzkonzentrationswert liegt, der in gesunden Personen auf eine hämostatische Stabilität hinweist.

13. Computerprogramm umfassend Programmcodemittel zum Veranlassen eines Computers, die Schritte des Verfahrens nach den Ansprüchen 1 bis 6 durchzuführen, wenn das genannte Computerprogramm auf dem Computer ausgeführt wird.

14. Computerlesbares nichtflüchtiges Speichermedium mit Anweisungen zur Ausführung durch einen Prozessor, wobei die Anweisungen den Prozessor veranlassen, die Schritte des Verfahrens nach den Ansprüchen 1 bis 6 durchzuführen.

**Revendications**

1. Procédé mis en oeuvre par ordinateur pour déterminer un risque hémostatique d'un sujet, le procédé comprenant une comparaison d'une valeur de concentration d'un déclencheur de coagulation suffisante pour démarrer le processus de coagulation chez un sujet avec au moins une valeur de concentration de référence dudit déclencheur de coagulation indicative d'un risque hémostatique et/ou d'un non-risque hémostatique (stabilité hémostatique), ledit procédé comprenant les étapes consistant à :

a) fournir une première information sur l'état hémostatique dudit sujet (S1), dans lequel ladite première information est les valeurs de concentration d'une pluralité de protéines de coagulation dans un échantillon de sang dudit sujet,

b) déterminer sur la base de ladite première information de l'étape (a) une valeur de concentration d'un déclencheur de coagulation suffisante pour démarrer le processus de coagulation chez ledit sujet (S2), dans lequel ledit déclencheur de coagulation est un facteur tissulaire, FT, dans lequel ladite valeur de concentration de FT suffisante pour démarrer le processus de coagulation chez le sujet est déterminée par une simulation *in silico* du processus de coagulation ; dans la simulation *in silico,* ladite première information sur l'état hémostatique dudit sujet est utilisée en tant que caractéristique d'entrée, et une seconde information sur l'état hémostatique dudit sujet dans le processus de coagulation simulé est utilisée en tant que caractéristique de sortie, dans lequel ladite seconde information sur l'état hémostatique dudit sujet est les valeurs de concentration d'une protéine de coagulation activée à une série de points temporels du processus de coagulation simulé qui sont utilisées en tant qu'ensemble de caractéristiques de sortie, et dans lequel la simulation *in silico* simule le changement dynamique des concentrations en protéine de coagulation après exposition à un FT,

c) comparer ladite valeur de concentration déterminée à l'étape (b) avec une valeur de concentration de référence dudit FT représentant chez des sujets de référence en bonne santé la concentration minimum pour un état hémostatique stable (S3), et

d) déterminer d'un risque élevé de thrombose pour ledit sujet si ladite valeur de concentration déterminée à l'étape (b) est inférieure à ladite valeur de concentration de référence dudit FT représentant chez des sujets de référence en bonne santé la concentration minimum pour un état hémostatique stable (S4).

2. Procédé selon la revendication 1, dans lequel les étapes c) et d) comprennent en outre les étapes suivantes :

   c') la comparaison de ladite valeur de concentration déterminée à l'étape (b) avec une valeur de concentration de référence dudit FT représentant chez des sujets de référence en bonne santé la concentration maximum pour un état hémostatique stable (S3'), et

   d') la détermination d'un risque élevé de saignement pour ledit sujet si ladite valeur de concentration déterminée à l'étape (b) est supérieure à ladite valeur de concentration de référence dudit FT représentant chez des sujets de référence en bonne santé la concentration maximum pour un état hémostatique stable (S4').

3. Procédé implémenté par ordinateur pour déterminer un risque hémostatique d'un sujet, le procédé comprenant une comparaison d'une valeur de concentration d'un déclencheur de coagulation suffisante pour démarrer le processus de coagulation chez un sujet avec au moins une valeur de concentration de référence dudit déclencheur de coagulation indicative d'un risque hémostatique et/ou d'un non-risque hémostatique (stabilité hémostatique), ledit procédé comprenant les étapes consistant à :

   a) fournir une première information sur l'état hémostatique dudit sujet (S1), dans lequel ladite première information est les valeurs de concentration d'une pluralité de protéines de coagulation dans un échantillon de sang dudit sujet,

   b) déterminer sur la base de ladite première information de l'étape (a) une valeur de concentration d'un déclencheur de coagulation suffisante pour démarrer le processus de coagulation chez ledit sujet (S2), dans lequel ledit déclencheur de coagulation est un FT, dans lequel ladite valeur de concentration de FT suffisante pour démarrer le processus de coagulation chez le sujet est déterminée par une simulation *in silico* du processus de coagulation ; dans la simulation *in silico,* ladite première information sur l'état hémostatique dudit sujet est utilisée en tant que caractéristique d'entrée, et une seconde information sur l'état hémostatique dudit sujet dans le processus de coagulation simulé est utilisée en tant que caractéristique de sortie, dans lequel ladite seconde information sur l'état hémostatique dudit sujet est les valeurs de concentration d'une protéine de coagulation activée à une série de points temporels du processus de coagulation simulé qui sont utilisées en tant qu'ensemble de caractéristiques de sortie, et dans lequel la simulation *in silico* simule le changement dynamique des concentrations en protéine de coagulation après exposition à un FT,

   c) comparer ladite valeur de concentration déterminée à l'étape (b) avec au moins deux valeurs de concentration de référence dudit FT comprenant au moins une valeur de concentration de référence indicatives chez des sujets de référence en bonne santé d'un risque hémostatique, de préférence un risque élevé de thrombose et/ou un risque élevé de saignement, et au moins une valeur de concentration de référence indicative chez des sujets de référence en bonne santé d'un non-risque hémostatique (stabilité hémostatique) (S3"), et

   d) déterminer

      - un risque hémostatique pour ledit sujet si ladite valeur de concentration déterminée à l'étape (b) est numériquement plus proche de ladite au moins une valeur de concentration de référence indicative chez des sujets de référence en bonne santé d'un risque hémostatique (S4"), ou

- un non-risque hémostatique (stabilité hémostatique) pour ledit sujet si ladite valeur de concentration déterminée à l'étape (b) est numériquement plus proche de ladite au moins une valeur de concentration de référence indicative chez des sujets en bonne santé d'une stabilité hémostatique (S4'''), ou

dans lequel, de préférence, les étapes (c) et (d) sont réalisées au moyen d'une approche du plus proche voisin.

4. Procédé selon la revendication 1, 2 ou 3, dans lequel, à l'étape (a), ladite première information sur l'état hémostatique dudit sujet est la valeur de concentration d'au moins trois protéines de coagulation ou plus dans un échantillon biologique dudit sujet, dans lequel en outre de préférence ladite ou lesdites protéines de coagulation sont choisies dans le groupe comprenant : facteur de coagulation 2 (FII), FV, FVII, FVIII, FIX, FX, FXI, FXII, antithrombine (AT), FTPI, $\alpha$2M, C4BP, protéine C, protéine S, protéine Z, TAFI, ZPI, AAT, PCI, inhibiteur de C1 et fibrinogène.

5. Procédé selon la revendication 1, dans lequel ladite seconde information sur l'état hémostatique dudit sujet est les valeurs de concentration d'une pluralité de protéines de coagulation activées à une série de points temporels du processus de coagulation simulé qui sont utilisées comme un ensemble de caractéristiques de sortie, dans lequel de la manière la plus préférée, ladite ou lesdites protéines de coagulation activées sont choisies dans le groupe comprenant : thrombine (FIIa), FVa, FVIIa, FVIIIa, FIXa, FXa, FXIIa, FVa-FXa, FVIIIa-FIXa, fibrine, prothrombine (FII).

6. Procédé selon la revendication 5, dans lequel hors de l'ensemble de caractéristiques de sortie, une caractéristique est créée, représentant la force de la réponse de coagulation, dans lequel de préférence ladite caractéristique représentant la force de la réponse de coagulation est la concentration maximum d'au moins une desdites protéines de coagulation activées à tous les points temporels du processus de coagulation simulé.

7. Dispositif pour déterminer le risque hémostatique d'un sujet, ledit dispositif comprenant :

une unité de réception configurée pour recevoir une première information sur l'état hémostatique dudit sujet, dans lequel ladite première information est les valeurs de concentration d'une pluralité de protéines de la coagulation dans un échantillon de sang dudit sujet,

une première unité de détermination configurée pour déterminer sur la base de ladite première information reçue par l'unité de réception une valeur de concentration d'un déclencheur de coagulation suffisante pour démarrer le processus de coagulation chez ledit sujet, dans lequel ledit déclencheur de coagulation est un FT, dans lequel ladite valeur de concentration de FT suffisante pour démarrer le processus de coagulation chez le sujet est déterminée par une simulation *in silico* du processus de coagulation, dans la simulation *in silico*, ladite première information sur l'état hémostatique dudit sujet est utilisée en tant que caractéristique d'entrée, et une seconde information sur l'état hémostatique dudit sujet dans le processus de coagulation simulé est utilisée en tant que caractéristique de sortie, dans lequel ladite seconde information sur l'état hémostatique dudit sujet est les valeurs de concentration d'une protéine de coagulation activée à une série de points temporels du processus de coagulation simulé qui sont utilisées en tant qu'ensemble de caractéristiques de sortie, et dans lequel la simulation *in silico* simule le changement dynamique des concentrations en protéine de coagulation après exposition à un FT, et

une unité de comparaison configurée pour comparer ladite valeur de concentration déterminée par ladite première unité de détermination avec au moins une valeur de concentration de référence dudit FT indicative chez des sujets de référence en bonne santé d'un risque hémostatique et/ou d'un non-risque hémostatique (stabilité hémostatique), dans lequel ladite unité de comparaison est configurée pour comparer ladite valeur de concentration déterminée par ladite première unité de détermination à une valeur de concentration de référence dudit FT représentant chez des sujets de référence en bonne santé la concentration minimum pour un état hémostatique stable, et ledit dispositif comprend en outre :

une seconde unité de détermination configurée pour déterminer un risque élevé de thrombose chez ledit sujet si ladite valeur de concentration déterminée par ladite première unité de détermination est inférieure à ladite valeur de concentration de référence dudit FT représentant chez des sujets de référence en bonne santé la concentration minimum pour un état hémostatique stable ;

dans lequel de préférence, en plus ou en variante,

ladite unité de comparaison est configurée pour comparer ladite valeur de concentration déterminée par ladite première unité de détermination avec une valeur de concentration de référence dudit FT représentant chez des sujets de référence en bonne santé la concentration maximum pour un état hémostatique stable, et

ladite seconde unité de détermination est configurée pour déterminer un risque élevé de saignement chez ledit sujet si ladite valeur de concentration déterminée par ladite première unité de détermination est supérieure à ladite valeur de concentration de référence dudit FT représentant chez des sujets de référence en bonne santé la concentration maximum pour un état hémostatique stable.

8. Dispositif selon la revendication 7, dans lequel ladite unité de comparaison est configurée pour comparer ladite valeur de concentration déterminée par ladite première unité de détermination avec au moins deux valeurs de concentration de référence ou plus dudit FT comprenant au moins une valeur de concentration de référence indicative chez des sujets de référence en bonne santé d'un risque hémostatique, de préférence un risque élevé de thrombose et/ou un risque élevé de saignement, et au moins une valeur de concentration indicative chez des sujets de référence en bonne santé d'un non-risque hémostatique (stabilité hémostatique), et ledit dispositif comprenant en outre :
une seconde unité de détermination configurée pour déterminer :

- un risque hémostatique chez ledit sujet si ladite valeur de concentration déterminée par ladite première unité de détermination est numériquement plus proche de ladite au moins une valeur de concentration de référence indicative chez des sujets de référence en bonne santé d'un risque hémostatique, ou
- un non-risque hémostatique (stabilité hémostatique) chez ledit sujet si ladite valeur de concentration déterminée par ladite première unité de détermination est numériquement plus proche de ladite au moins une valeur de concentration de référence indicative chez des sujets de référence en bonne santé d'une stabilité hémostatique.

9. Système d'aide à la décision clinique comprenant un processeur et un support de stockage lisible par ordinateur, dans lequel ledit support de stockage lisible par ordinateur contient des instructions à exécuter par le processeur, dans lequel lesdites instructions amènent ledit processeur à effectuer les étapes consistant à :

a) recevoir une première information sur l'état hémostatique d'un sujet, ladite première information étant les valeurs de concentration d'une pluralité de protéines de la coagulation dans un échantillon de sang dudit sujet,
b) déterminer sur la base de ladite information de l'étape (a) une valeur de concentration d'un déclencheur de coagulation suffisante pour démarrer le processus de coagulation chez ledit sujet, dans lequel ledit déclencheur de coagulation est un facteur tissulaire, FT, dans lequel ladite valeur de concentration de FT suffisante pour démarrer le processus de coagulation chez le sujet est déterminé par une simulation *in silico* du processus de coagulation, dans la simulation *in silico*, ladite première information sur l'état hémostatique dudit sujet est utilisée comme caractéristique d'entrée et une seconde information sur l'état hémostatique dudit sujet. dans le processus de coagulation simulé est utilisé comme caractéristique de sortie, ladite seconde information sur l'état hémostatique dudit sujet étant les valeurs de concentration d'une protéine de coagulation activée à une série de moments du processus de coagulation simulé qui sont utilisés en tant qu'ensemble de sortie et dans laquelle la simulation *in silico* simule le changement dynamique de la concentration en protéines de la coagulation après exposition au FT,
c) comparer ladite valeur de concentration déterminée à l'étape (b) avec au moins une valeur de concentration de référence dudit FT indicative chez des sujets de référence en bonne santé pour un risque hémostatique et/ou un non-risque hémostatique (stabilité hémostatique).

10. Système d'aide à la décision clinique comprenant un processeur et un support de stockage lisible par ordinateur, dans lequel ledit support de stockage lisible par ordinateur contient des instructions à exécuter par le processeur, lesdites instructions forçant ledit processeur à effectuer les étapes consistant à :

a) fournir une première information sur l'état hémostatique dudit sujet, dans lequel ladite première information est les valeurs de concentration d'une pluralité de protéines de coagulation dans un échantillon de sang dudit sujet,
b) déterminer sur la base de ladite première information de l'étape (a) une valeur de concentration d'un déclencheur de coagulation suffisante pour démarrer le processus de coagulation chez ledit sujet, dans lequel ledit déclencheur de coagulation est un FT, dans lequel ladite valeur de concentration de FT suffisante pour démarrer le processus de coagulation chez le sujet est déterminée par une simulation *in silico* du processus de coagulation ; dans la simulation *in silico,* ladite première information sur l'état hémostatique dudit sujet est utilisée en tant que caractéristique d'entrée, et une seconde information sur l'état hémostatique dudit sujet dans le processus de coagulation simulé est utilisée en tant que caractéristique de sortie, dans lequel ladite seconde information sur l'état hémostatique dudit sujet est les valeurs de concentration d'une protéine de coagulation activée à une série de points temporels du processus de coagulation simulé qui sont utilisées en tant qu'ensemble de caractéristiques de sortie, et dans lequel la simulation *in silico* simule le changement dynamique des concentrations en protéine de coagulation après exposition à un FT,

c) comparer ladite valeur de concentration déterminée à l'étape (b) avec une valeur de concentration de référence dudit FT représentant chez des sujets de référence en bonne santé la concentration minimum pour un état hémostatique stable et représentant, chez des sujets de référence en bonne santé, la concentration minimale pour un état hémostatique stable, et

d) déterminer un risque élevé de thrombose chez ledit sujet si ladite valeur de concentration déterminée à l'étape (b) est inférieure à ladite valeur de concentration de référence dudit FT représentant chez des sujets de référence en bonne santé la concentration minimum pour un état hémostatique stable.

**11.** Système d'aide à la décision clinique selon la revendication 9 ou 10, dans lequel les étapes c) et d) comprennent en outre les étapes suivantes :

c') la comparaison de ladite valeur de concentration déterminée à l'étape (b) avec une valeur de concentration de référence dudit FT représentant dans des sujets de référence en bonne santé la concentration maximum pour un état hémostatique stable, et

d') la détermination d'un risque élevé de saignement chez ledit sujet si ladite valeur de concentration déterminée à l'étape (b) est supérieure à ladite valeur de concentration de référence dudit FT représentant chez des sujets de référence en bonne santé la concentration maximum pour un état hémostatique stable.

**12.** Système d'aide à la décision clinique comprenant un processeur et un support de stockage lisible par ordinateur, dans lequel ledit support de stockage lisible par ordinateur contient des instructions à exécuter par le processeur, dans lequel lesdites instructions amènent ledit processeur à effectuer les étapes consistant à :

a) fournir une première information sur l'état hémostatique dudit sujet, dans lequel ladite première information est une valeur de concentration d'une pluralité de protéines de coagulation dans un échantillon de sang dudit sujet,

b) déterminer sur la base de ladite première information de l'étape (a) une valeur de concentration d'un déclencheur de coagulation suffisante pour démarrer le processus de coagulation chez ledit sujet, dans lequel ledit déclencheur de coagulation est un FT, dans lequel ladite valeur de concentration de FT suffisante pour démarrer le processus de coagulation chez le sujet est déterminée par une simulation *in silico* du processus de coagulation, dans la simulation *in silico*, ladite première information sur l'état hémostatique dudit sujet est utilisée en tant que caractéristique d'entrée, et une seconde information sur l'état hémostatique dudit sujet dans le processus de coagulation simulé est utilisée en tant que caractéristique de sortie, dans lequel ladite seconde information sur l'état hémostatique dudit sujet est les valeurs de concentration d'une protéine de coagulation activée à une série de points temporels du processus de coagulation simulé qui sont utilisées en tant qu'ensemble de caractéristiques de sortie, et dans lequel la simulation *in silico* simule le changement dynamique des concentrations en protéine de coagulation après exposition à un FT,

c) comparer ladite valeur de concentration déterminée à l'étape (b) à au moins deux valeurs de concentration de référence ou plus dudit FT comprenant au moins une valeur de concentration de référence indicative chez des sujets de référence en bonne santé d'un risque hémostatique, de préférence un risque élevé de thrombose et/ou un risque élevé de saignement, et au moins une valeur de concentration indicative chez des sujets de référence en bonne santé d'un non-risque hémostatique (stabilité hémostatique), et

d) déterminer :

- un risque hémostatique chez ledit sujet si ladite valeur de concentration déterminée à l'étape (b) est numériquement plus proche de ladite au moins une valeur de concentration de référence indicative chez des sujets de référence en bonne santé d'un risque hémostatique, ou

- un non-risque hémostatique (stabilité hémostatique) chez ledit sujet si ladite valeur de concentration déterminée à l'étape (b) est numériquement plus proche de ladite au moins une valeur de concentration de référence indicative chez des sujets de référence en bonne santé d'une stabilité hémostatique.

**13.** Programme informatique comprenant un moyen de code de programme pour amener un ordinateur à exécuter les étapes du procédé des revendications 1 à 6 lorsque ledit programme informatique est exécuté sur l'ordinateur.

**14.** Support de stockage non transitoire lisible par ordinateur contenant des instructions à exécuter par un processeur, dans lequel les instructions amènent le processeur à exécuter les étapes du procédé des revendications 1 à 6.

FIG.1

FIG.2

FIG.3

FIG.4

FIG.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090298103 A

- WO 2009142744 A

### Non-patent literature cited in the description

- **KYRLE PA ; EICHINGER S.** Deep vein thrombosis. *Lancet,* 2005, vol. 365 (9465), 1163-74
- **ENGBERS MJ ; VAN HYLCKAMA VLIEG A ; ROSENDAAL FR.** Venous thrombosis in the elderly: incidence, risk factors and risk groups. *J Thromb Haemost,* 2010, vol. 8 (10), 2105-12
- **CANNEGIETER SC ; DOGGEN CJ ; VAN HOUWELINGEN HC ; ROSENDAAL FR.** Travel-related venous thrombosis: results from a large population-based case control study (MEGA study). *PLoS Med.,* 2006, vol. 3 (8), e307
- **KEARON C ; KAHN SR ; AGNELLI G ; GOLDHABER S ; RASKOB GE ; COMEROTA AJ.** American College of Chest Physicians. Antithrombotic therapy for venous thromboembolic disease: American College of Chest Physicians Evidence-Based Clinical Practice Guidelines. *Chest,* June 2008, vol. 133 (6), 454S-545S
- **VEEGER NJ ; PIERSMA-WICHERS M ; TIJSSEN JG ; HILLEGE HL ; VAN DER MEER J.** Individual time within target range in patients treated with vitamin K antagonists: main determinant of quality of anticoagulation and predictor of clinical outcome. A retrospective study of 2300 consecutive patients with venous thromboembolism. *Br J Haematol,* 2005, vol. 128 (4), 513-9

- **COHEN AT ; TAPSON VF ; BERGMANN JF ; GOLDHABER SZ ; KAKKAR AK ; DESLANDES B ; HUANG W ; ZAYARUZNY M ; EMERY L ; ANDERSON FA JR.** ENDORSE Investigators. Venous thromboembolism risk and prophylaxis in the acute hospital care setting (ENDORSE study): a multinational cross-sectional study. *Lancet,* 2008, vol. 371 (9610), 387-94
- **WHITE RH.** The epidemiology of venous thromboembolism. *Circulation,* 2003, vol. 107 (1), 14-8
- **BRUMMEL-ZIEDINS KE ; ORFEO T ; ROSENDAAL FR ; UNDAS A ; RIVARD GE ; BUTENAS S ; MANN KG.** Empirical and theoretical phenotypic discrimination. *J Thromb Haemost,* 2009, vol. 7 (1), 181-6
- **HEMKER, HC ; BÉGUIN, S.** Thrombin generation in plasma: Its assessment via the endogenous thrombin potential. *Thrombosis and haemostasis,* 1995, vol. 74 (1), 134-138
- **JORDAN, SW ; CHAIKOV, EL.** Simulated Surface-Induced Thrombin Generation in a Flow Field. *Biophysical Journal,* July 2011, vol. 101, 276-286
- The Dynamics of Thrombin Formation. **MANN, KG ; BUTENAS, S. ; BRUMMEL, K.** Arterioscler. Thromb. Vasc. Biol. 2003, vol. 23, 17-25
- **HIPPISLEY-COX, J ; COUPLAND, C.** Development and validation of risk prediction algorithm (QThrombosis) to estimate future risk of venous thromboembolism: prospective cohort study. *BMJ,* 2011, 343